# FASCICULE DE BREVET EUROPEEN

(11) **EP 3 749 281 B1**
(45) Date de publication et mention de la délivrance du brevet: **26.06.2024**
(21) Numéro de dépôt: 19702464.9
(22) Date de dépôt: 07.02.2019
(51) Int. Cl.: A61Q 13/00, A61K 8/34, A61K 8/39, C11D 1/72, C11D 3/50, C11D 17/00

(54) **COMPLEXE PARFUMANT ET COMPOSITION D'EAU PARFUMÉE**
PARFÜMZUBEREITUNG UND ZUBEREITUNG AUS PARFÜMIERTEM WASSER
PERFUMING COMPLEX AND COMPOSITION OF PERFUMED WATER

(30) Priorité: 09.02.2018 FR 1851138; 10.09.2018 FR 1858100
(43) Date de publication de la demande: 16.12.2020
(73) Titulaire: Expressions Parfumees, 06130 Grasse (FR)
(72) Inventeur: MARIN, Christophe, 06200 NICE (FR); BUZZI, Jennifer, 06130 GRASSE (FR)
(74) Mandataire: Hautier IP
(86) Numéro de dépôt international: PCT/EP2019/052967
(87) Numéro de publication internationale: WO 2019/154892

(56) Documents cités:
- EP-A1- 1 051 148
- EP-A1- 2 471 506
- WO-A1-2014/187950
- DE-A1-102009 060 360
- US-A- 6 110 449
- US-A1- 2013 280 175
- US-A1- 2014 315 772

## Description

### Domaine de l'invention

La présente invention a pour objet un complexe parfumant ne comprenant pas d'eau et comprenant trois composants de base destinés à former avec un milieu aqueux une composition et une composition d'eau parfumée avantageusement sprayable comprenant du parfum, de l'isopentyldiol et du C12-13 pareth-9, avantageusement sous forme de micro-émulsion avantageusement sans éthanol, avantageusement à haut dosage de parfum et avantageusement transparente.

### L'art antérieur

Dans le domaine de la parfumerie, un problème récurrent concerne la solubilité des parfums dans l'eau.

Pour assurer à la fois une solubilité et une performance olfactive suffisantes à l'utilisateur, il s'est avéré nécessaire d'ajouter certains additifs. De tels additifs présentent notamment l'inconvénient de laisser un toucher gras et collant et pour la plupart d'irriter la peau. Ils modifient également les propriétés de la solution qui les contient comme la viscosité et la proportion de mousse formée.

Des recherches ont porté sur l'utilisation de plusieurs solubilisants et co-solvants, notamment des diols vicinaux, peu performants pour des dosages de parfum élevés, tels que le 1,2-hexanediol. De tels composés présentent pour certains l'inconvénient d'être très moussants, très collants et doivent être chauffés pour être utilisés.

De nombreuses compositions actuellement commercialisées contiennent de l'éthanol. Or, l'éthanol est un COV (Composé Organique Volatil) ce qui est dommageable vis-à-vis de certaines contraintes réglementaires et environnementales. Parallèlement, des recherches ont permis de démontrer que la présence d'eau et la diminution voire l'absence de l'éthanol permettaient d'augmenter la ténacité du parfum dans le temps, d'éviter l'irritation et l'assèchement de la peau et des cheveux, de s'affranchir des risques liés à une exposition au soleil et d'éviter tout problème lié au stockage, transport (douanes) et à l'inflammabilité.

Afin de remédier aux inconvénients précités, il a été proposé des compositions à base de co-solvants tels que l'isosorbide, le solketal, et des éthers de ceux-ci, comme dans le document WO2014/187950 qui utilise en plus d'un de ces co-solvants du Barsolve Plus, mélange de 40-60% Trideceth-9, 20-40% PEG-40 Hydrogenated Castor oil, 5% Polysorbate-20, solubilisant commercialisé par Barnet. Il a également été proposé des compositions comprenant de l'alcool oléylique éthoxylé (commercialisé sous le nom d'Ameroscol^{®}) ou de sulfates, tels que le sodium lauryl sulfate et le sodium laureth sulfate, en tant que tensioactifs. Cependant, de tels composés sont irritants et asséchants pour la peau et les cheveux et sont donc à éviter dans le domaine de la parfumerie et de la cosmétique.

En parfumerie, l'obtention d'un mélange odorant qui soit à la fois polyvalent en termes d'applications, stable, non-irritant pour la peau et qui présente des propriétés sensorielles améliorées, fait l'objet de recherches permanentes.

### Résumé de l'invention

L'invention concerne un complexe parfumant ne comprenant pas d'eau et comprenant trois composants de base suivants : 15 à 45 % en poids de parfum, 15 à 50 % d'isopentyldiol en poids et 15 à 30 % en poids de C12-13 pareth-9, les pourcentages étant exprimés par rapport au poids total des trois composants de base.

Le complexe parfumant selon l'invention présente l'avantage de ne pas contenir d'eau, ce qui facilite son transport, son conditionnement tout en étant stable et non irritant pour la peau.

Avantageusement, le complexe parfumant ne comprend pas d'éthanol.

L'invention concerne l'utilisation du complexe parfumant dans un milieu aqueux.

En effet, le complexe parfumant est destiné à être utilisé dans un milieu aqueux pour former notamment une composition.

Suivant un autre aspect, l'invention concerne une composition comprenant de l'eau et le complexe parfumant selon l'invention, et ne contenant aucun co-solvant du groupe comprenant l'isosorbide, le solketal et des éthers de ceux-ci.

Selon un mode de réalisation, la composition est exempte d'éthanol.

Selon un mode de réalisation, la composition comprend de 30 à 40 % de complexe parfumant.

Selon un autre mode de réalisation, l'invention concerne une composition comprenant 5 à 40 % en poids de parfum, 5 à 30 % en poids C12-13 pareth-9pareth-9, et 5 à 30 % en poids d'isopentyldiol, les pourcentages étant exprimés par rapport au poids total de ladite composition.

Elle porte également sur une base cosmétique, base détergente ou parfum d'ambiance comprenant ladite composition, avantageusement la composition est une base cosmétique, une base détergente ou un parfum d'ambiance.

Elle porte également sur l'utilisation de ladite composition, de ladite base cosmétique, de ladite base détergente et dudit parfum d'ambiance pour masquer les mauvaises odeurs.

### Descriptif détaillé

La présente invention a ainsi pour objet un complexe parfumant avantageusement pour obtenir une composition et une nouvelle composition aqueuse parfumée avantageusement sprayable qui soient :
(1) peu collante et peu moussante,
(2) compatible avec n'importe quel type de famille olfactive,
(3) à haut dosage de parfum,
(4) qui ne s'évapore pas trop rapidement, et
(5) qui présente une solubilité et une performance olfactive suffisantes.

En effet, ces cinq critères peuvent être contradictoires. Or, les présents inventeurs ont eu le mérite de trouver un complexe parfumant pour obtenir une composition et une composition aqueuse parfumée avantageusement sprayable qui présentent un excellent compromis entre ces différents critères (1)-(5).

Le complexe parfumant selon l'invention assure un équilibre entre le C12-13 pareth-9 et l'isopentyldiol. En effet, la formulation du complexe parfumant sans eau en vue de son mélange avec de l'eau doit satisfaire des contraintes de stabilité du complexe mais également les contraintes de la composition mentionnées ci-dessus. Les inventeurs ont défini que :
- un complexe parfumant contenant moins de 15 % en poids de parfum ne permettra pas d'obtenir une composition aqueuse parfumée suffisamment puissante,
- un complexe parfumant contenant plus de 45 % en poids de parfum ne permettra pas d'obtenir une composition aqueuse parfumée transparente quelle que soit la famille olfactive,
- un complexe parfumant comprenant moins de 15 % en poids de C12-13 pareth-9 ne permet pas de solubiliser un complexe parfumant comprenant 15 % en poids de parfum et d'obtenir une composition aqueuse parfumée transparente et puissante,
- un complexe parfumant comprenant plus de 30 % en poids de C12-13 pareth-9 induit une nouvelle composition aqueuse parfumée trop collante et moussante,
- un complexe parfumant comprenant moins de 15 % en poids d'isopentydiol ne permet pas de solubiliser un complexe parfumant comprenant 15 % en poids de parfum et d'obtenir une composition aqueuse parfumée transparente
- un complexe parfumant comprenant moins de 15 % en poids d'isopentydiol ne permet pas d'obtenir une composition aqueuse parfumée au toucher peu collant.
- un complexe parfumant comprenant plus de 50 % en poids d'isopentydiol ne permet pas d'obtenir à la fois un haut dosage de parfum et un dosage de C12-13 Pareth-9 nécessaire à la solubilisation en vue d'une composition aqueuse transparente.

En outre, ladite composition mise au point par les présents inventeurs est avantageuse puisqu'elle peut être sans éthanol, transparente, stable, peu moussante, non irritante pour la peau, agréable une fois appliquée sur la peau, présente une viscosité proche de celle de l'eau, présente de bonnes propriétés de séchage, présente une tenue et perception olfactive suffisantes, et/ou présente des propriétés sensorielles améliorées.

Par « transparente », on entend que ladite composition est transparente pour des températures comprises entre 4 et 50°C quel que soit le parfum utilisé. Par composition transparente, on entend une composition dont la turbidité est inférieure à 7 NTU (Nephelometric Turbidity Unit). Celle-ci étant préférentiellement mesurée à l'aide d'un turbidimètre de type HI88713 Iso Turbidimeter de la marque HANNA Instruments.

Par « sans-éthanol », on entend une composition contenant 0 % en poids d'alcool éthylique.

Par « stable » on entend une composition qui ne se dégrade pas avec le temps et dont les composés ne vont pas réagir entre eux. Par « stable » on entend que l'homogénéité, la transparence, l'odeur agréable et la viscosité de la composition sont conservées. Plus particulièrement, la composition selon la présente invention est stable en vieillissement accéléré après 24h aux UV (Suntest) et après 2 mois à 25°C et 45°C en comparaison avec un échantillon resté à 5°C, à l'abri de la lumière.

Par « présentant une viscosité proche de celle de l'eau » on entend que la viscosité dynamique à 20°C de ladite composition est comprise entre 1 et 50 mPa.s, préférentiellement entre 1 et 40 mPa.s, et encore plus préférentiellement entre 1 et 30 Pa·s. Selon la présente invention, la viscosité dynamique est mesurée avec le viscosimètre Viscotester IQ, géométrie CC27 DG/TI - 01160025, à 20°C, à 500 s⁻¹ pendant 30 secondes.

Par « bonnes propriétés de séchage » on entend qu'il est possible d'obtenir un toucher sec à l'étalement au bout de quelques minutes, préférentiellement au bout d'une minute, et encore plus préférentiellement au bout de quelques secondes.

Par « compatible avec n'importe quel type de famille olfactive », on entend que ladite composition peut contenir n'importe quel type de parfum.

Par « haut dosage de parfum », on entend que la composition peut contenir jusqu'à 20 %, jusqu'à 30 % et jusqu'à 45 % en poids de parfum.

Le complexe selon l'invention ne comprend pas d'eau et comprend les trois composants de base suivants : 15 à 45 % en poids de parfum, 15 à 50 % en poids d'isopentyldiol et 15 à 40 %, préférentiellement 30 %, en poids de C12-13 pareth-9, les pourcentages étant exprimés par rapport au poids total des 3 composants de base.

Le complexe est une forme concentrée de composition. Le complexe ne comprend avantageusement pas d'éthanol. Préférentiellement, le complexe est constitué des trois composants de base.

Selon un mode de réalisation, le complexe comprend de 20 à 40 % en poids de parfum, plus préférentiellement de 27 à 31 % en poids de parfum et à titre d'exemple préféré 28 % en poids de parfum.

Selon un mode de réalisation, le complexe comprend de 20 à 30 % en poids de C12-13 pareth-9, plus préférentiellement de 24 à 29 % en poids de C12-13 pareth-9, et à titre d'exemple préféré 26 % en poids de C12-13 pareth-9.

Selon un mode de réalisation, le complexe comprend de 35 à 50 % en poids d'isopentyldiol, plus préférentiellement de 42 à 47 % en poids d'isopentyldiol et à titre d'exemple préféré 46 % en poids d'isopentyldiol.

Avantageusement, le complexe selon l'invention n'est pas destiné à être utilisé tel quel mais uniquement vendu pour faciliter le transport et le conditionnement. Préférentiellement, le complexe est destiné à être mélangé à au moins un solvant aqueux, de préférence à au moins de l'eau pour former une composition selon l'invention.

Selon un mode de réalisation, la composition comprend de 30 à 40 % de complexe parfumant, préférentiellement 35 % en poids du poids total de composition.

Selon un mode de réalisation, la composition selon l'invention est une composition comprenant 5 à 40 % en poids de parfum, 5 à 30 % en poids C12-13 pareth-9, et 5 à 30 % en poids d'isopentyldiol, les pourcentages étant exprimés par rapport au poids total de ladite composition.

La composition selon la présente invention est une composition parfumée.

Par « composition parfumée », on entend une composition comprenant un mélange de substances parfumantes, lesdites substances parfumantes étant à l'état isolé, en solution ou en suspension, dans leurs diluants, dissolvants ou co-ingrédients habituels. Une telle composition est destinée à apporter une composante olfactive agréable.

Par « parfum » ou « substance parfumante », on entend une ou plusieurs matières premières naturelles ou synthétiques parfumées.

De telles matières premières, qu'elles soient naturelles ou synthétiques, peuvent comprendre des esters, éthers, alcools, aldéhydes, cétones, lactones, acétals, nitriles, phénols, acides, terpènes, composés hétérocycliques azotés ou soufrés, saturés ou insaturés, et des produits complexes d'origine naturelle.

Des exemples d'esters comprennent, mais ne sont pas limités à, l'acétate de benzyle, acétate de p-tert-butylcyclohexyle, 3,7-diméthyl-1,6-octadien-3-yl acétate (acétate de linalyle), acétate de diméthyl-benzyl-carbinyle, acétate de phényléthyle, acétate de 1,1-diméthyl-2-phényléthyle, benzoate de linalyle, glycinate d'éthyl-méthyl-phényle, propionate d'allylcyclohexyle, propionate de styrallyle, salicylate de benzyle, acétate de méthyl-3-oxo-2-pentylcyclopentane, acétate de prop-2-ènyle-2,3-méthylbutoxy (glycolate d'allyl amyle, ester 2-propénylique de l'acide 3-méthylbutoxy-acétique), ester phénylméthylique de l'acide acétique, acétate d'isoamyle (acétate d'isopentyle), acétate de cis-hex-3-ènyle (acétate de (Z)-hex-3-ènyle), acétate de citronellyle (acétate de 3,7-dimethyl-6-octèn-1-ol), acétate d'hexyle, acétate d'isobornyle (exo-acétate de bicyclo[2.2.1]heptan-2-ol,1,7,7-triméthyle), acétate de méthanyle (acétate d'alpha,alpha,4-triméthylcyclohexylméthyle), acétate d'éthyle, acétate de prényle (acétate de 3-méthyl-2-butényle), citrate de triéthyle, acétate de 4-ter-Butylcyclohexyle, acétate de (3R-(3alpha,3abeta,6alpha,7beta,8aalpha))-Octahydro-3,6,8,8-tetraméthyl-1H-3a-7-méthanoazulèen-5-yl, acétate de 3,7-Diméthyl octa-1,6-diène-3-yl, 1,4-Dioxacyclohexadecane-5,16-Dione, 2-hydroxybenzoate de benzyle, (Z)-3-Hexenyl-2-hydroxybenzoate, acétate de 2-(1,1(Diméthyléthyl) Cyclohexyle, acétate d'isopentyl, Méthyl Phénylacétate, acétate de (Z)-Hex-3-enyl, acétate de 3,7-Diméthyl octa-1,6-diene-3-yl, acétate de 3-Méthyl-2-butényl, acétate de alpha-Méthyle-Benzèneméthanol, 2-aminobenzoate de méthyle, 2-Propényl-(cyclohexyloxy)acétate, 2,4-dihydroxy-3,6-diméthylbenzoate de méthyle, 3-oxo-2-pentylcyclopentaneacétate de méthyle, Propanoate de 3-alpha,4,5,6,7,7-alpha-Hexahydro-4,7-methano-1H-inden-6-yl, méthyl 3-oxo-2-pentylcyclopentaneacétate, Acétate 2-(1,1-Diméthyléthyl)Cyclohexyl, hexyl-2-hydroxybenzoate (2-hydroxy-2-hexyl ester d'acide benzoïque), acétate de 3a,4,5,6,7,7a-Hexahydro-4,7-méthanoinden-6-yl, 2-méthylbutyrate d'éthyle, 3a,4,5,6,7,7a-Hexahydro-4,7-méthano-1H-inden-5-yl, 2-méthylpropanoate, Acétate de alpha,alpha-Diméthylphénéthyl, Acétate de 3,7-Diméthyl octa-1,6-diene-3-yl, Acétate d'exo-1,7,7-triméthyl-bicyclo[2.2.1]heptan-2-ol, hexanoate d'éthyle, acétate de 3,7-Diméthyl octa-1,6-diene-3-yl, méthyl 3-oxo-2-pentylcyclopentaneacétate, Méthyl 2-(méthylamino)benzoate, Acide 10-Undécenoïque, ester d'éthyle, 2-méthylpentanoate d'éthyle, éthanoate de cis-3,7-Diméthyl-2,6-octadienyle, 2-hydroxybenzoate de benzyle, acétate de (Z)-Hex-3-enyle, 2-hydroxy-2-hexyl ester d'acide benzoïque, prop-2-ènyl-2-cyclohexyloxyacetate (2-Propényl-(cyclohexyloxy)acétate), Acide acétique, (3-Methylbutoxy), Ester de 2-Propényle, 2-Phényléthanol, Acétate d'hexyle, (1R, 2S, 5R)-5-Méthyl-2-(1-méthyléthyl)-cyclohexanol éthanoate, acétate de terpényle (acétate de 4-méthyl-1-propan-2-yl-1-cyclohex-2-ènyle), formiate d'alpha-3,3-triméthylcyclohexyle-méthyle, butanoate de 3-méthylbutyle (butyrate d'iso amyle), butanoate d'alpha,alpha-diméthylphenethyl, acétate de 3-dihydrodicyclopentadièn-2,3-yle, prop-2-ènyl, propanoate de 3-cyclohexyle (cyclo hexane propionate d'allyl), heptanoate d'allyle (heptanoate de 2-propényl), 2-methylpropanoate de 2-phénoxy-éthyle (isobutyrate de phénoxy éthyle), 2-méthylpentanoate d'éthyle, 2-méthyl-butyrate d'éthyle (ester éthylique de l'acide 2-méthyl-butanoïque), 1,4-dioxacycloheptadecane-5,17-dione (Brassylate d'éthylène), propanoate de (2S)-2-propyl-1,1-dimethyl-propoxy ((2S)-Ester propylique de l'acide 2-(1,1-diméthylpropoxy)-propanoïque), acétate de 2-tert-butylcyclohexyle (acétate de 2-(1,1-diméthylethyl)cyclohexyle), salicylate de ci-3-hexenyle, acétate de [(1S)-3-(4-méthylpent-3-ènyl)-1-cyclohex-3-ènyl]méthyle, l'acétate de 3-pentyltetrahydro[2H]pyranyle, propionate de linalyle, acétate de cétyle, acétate de cédryle, acétate d'anisyle, acétate de nopyle, acétate de néryle, acétate de 3a,4,5,6,7,7a-hexahydro-4,7-methanoindèn-6-yle, propanoate de 3a,4,5,6,7,7a-hexahydro-4,7-methano-1H-indèn-6-yl, 3-cyclohexanepropanoate de 2-propényl, 2-hydroxypropane-1,2,3-tricarboxylate de 1,2,3-triéthyle, acétate de (2E)-3,7-diméthylocta-2,6-dièn-1-yle, acétate de 3,5,5-trimethylhexyle, acétate de 3,7-diméthyl-octa-1,6-diene-3-yle, éthanoate de cis-3,7-diméthyl-2,6-octadiènyle, ester de 1-méthyléthyl de l'acide tetradécanoïque, ester 3-méthylbutylique de l'acide 2-hydroxy-benzoïque, ester phénylméthylique de l'acide 2-hydroxy-benzoïque, ester 2-hexylique de l'acide 2-hydroxy-benzoïque, ester méthylique de l'acide 2-hydroxy-Benzoïque, ester éthylique de l'acide acétoacétique, acétate de 3,7-diméthyl-octa-1,6-diène-3-yle, ester 1,2-diéthylique de l'acide 1,2-benzènedicarboxylique, 2-méthylpropanoate de (Z)-hex-3-ènyle, acétate de (4-méthyl-1-propan-2-yl-1-cyclohex-2-enyl), acétate de 3a,4,5,6,7,7a-hexahydro-4,7-méthanoindèn-6-yle, 2,3-époxy-3-phénylbutyrate d'éthyle, 2-aminobenzoate de méthyle, 2-(méthylamino)benzoate de méthyle, benzoate de méthyle, 2,4-dihydroxy-3,6-diméthylbenzoate de méthyle, acétate (3R-(3alpha,3beta,6beta,7beta,8alpha))-octahydro-6-méthoxy-3,6,8,8-tetraméthyl-1H-3a,7-méthanoazulène, salicylate d'hexyle, 4-tert-butylcyclohexyl) acétate, palmitate de méthyle, 1,6-octadieèn-3-ol, 3,7-diméthyl-acétate et le citrate de triéthyle. De manière préférée, des exemples d'esters comprennent le propionate de linalyle, acétate de cétyle, acétate de cédryle, acétate d'anisyle, acétate de nopyle, acétate de néryle, acétate (3R-(3alpha,3beta,6beta,7beta,8alpha))-octahydro-6-méthoxy-3,6,8,8-tetraméthyl-1H-3a,7-méthanoazulène, salicylate d'hexyle, 4-tert-butylcyclohexyl) acétate, palmitate de méthyle, 1,6-Octadien-3-ol, 3,7-dimethyl-acétate, acétate de linalyle et le citrate de triéthyle.

Des exemples d'éthers comprennent, mais ne sont pas limités à, l'éther de benzyle, éther éthylique, ambre gris, oxyde de diphényle, 4,6,6,7,8,8-hexaméthyl-1,3,4,6,7,8-hexahydrocyclopenta[g]isochromene, ambre carane, 1,1-diméthoxy-2,2,5-triméthyl-4-hexène, 3,4,4a,5,8,8a-Hexahydro-3',7'-diméthylspiro(1,4-methanonaphthalène-2(1H),2'-oxirane), 2,4,6-Triméthyl-4-phényl-1,3-dioxane, Benzène, 1,1'-Oxybis, Méthyle 2-naphthyl éther, éthyle 2-naphthyl éther, 2,4-Diméthyl-4-phényltetrahydrofurane, (éthoxyméthoxy)cyclododecane, (E)-1-méthoxy-4-(1-propényl)-benzène, 1-méthoxy-4-(2-propenyl)-benzène, Méthyl cédryl éther et l'éther éthylique de 2-naphtyle. De manière préférée, des exemples d'éthers comprennent, le 1,1-diméthoxy-2,2,5-triméthyl-4-hexène, Méthyl cédryl éther et l'éther éthylique de 2-naphtyle.

Des exemples d'alcools comprennent, mais ne sont pas limités à, le menthol ([1R-(1alpha,2beta,5alpha)]-5-méthyl-2-isopropylcyclohexanol), citronellol, géraniol, linalol (par exemple l'éthyle linalol et le tetrahydro linalol), alcool phényléthylique, terpinéol, 2,6-diméthylheptan-2-ol, 2-méthyl-1-phénylpropan-2-ol (diméthyl phényle carbinol), 3-méthyl-5-[2,2,3-triméthylcyclopent-3-èn-1-yl]pent-4-èn-2-ol, 2-phényléthanol, 2-éthyl-4-(2,2,3-triméthyl-1-cyclopent-3-enyl)but-2-èn-1-ol, (E)-4-méthyldec-3-èn-5-ol, alcool cinnamique (3-phényl-2-propèn-1-ol), 3,7-Diméthyl-6-octen-1-ol, p-menth-1-en-8-ol, cis-Hex-3-en-1-ol, 4-Méthyl-3-decen-5-ol, 2,6-diméthyloct-7-en-2-ol, 1,1'-Oxydipropan-2-ol, 3,7-Diméthyl-1,6-nonadien-3-ol, 1,1'-Oxydipropan-2-ol, 2,6-diméthyloct-7-en-2-ol 3,7-Diméthyl octa-1,6-diene-3-ol, 2-éthyl-4-(2,2,3-triméthyl-3-cyclopenten-1-yl)-2-buten-1-ol, phénylméthanol, 4-Méthyl-3-decen-5-ol, 2,6-Diméthyloctan-2-ol, 3,7-Diméthyl-6-octen-1-ol, 4-(1,1-diméthyléthyl)-Cyclohexanol, , (2E)-3,7-diméthyl-2,6-Octadien-1-ol, Hexan-1-ol, exo-1,7,7-Triméthylbicyclo[2.2.1]heptan-2-ol, 2-(2,2,7,7-tetraméthyltricyclo[6.2.1.0 (1,6)]undec-5(4)-en-5-yl)propan-1-ol, 1-Phenyléthanol, 1,1'-Oxydipropan-2-ol, 3,7-Diméthyloctan-3-ol, cis-3,7-Diméthyl-2,6-octadien-1-ol, 3,7-Diméthyl-6-octen-1-ol, Hex-2-en-1-ol, 3,7-Diméthyl octa-1,6-diène-3-ol, 2-Méthyl-4-phénylbutan-2-ol, 2,6-Octadien-1-ol, 3,7-diméthyl-, (2E)-, p-menth-1-en-8-ol, 3-Phénylpropan-1-ol, phénylméthanol, 2,6-diméthyloct-7-èn-2-ol, alpha,beta,2,2,3-pentaméthylcyclopent-3-ène-1-butanol, 3-(5,5,6-triméthylbicyclo[2.2.1]hept-2-yl) cyclohexan-1-ol (IBCH), cis-3-hexèn-1-ol, méthyl-triméthylbicyclo-hexylméthyl-cyclopropyl méthanol alcool benzylique, endo-1,7,7-triméthyl-bicyclo-[2.2.1]heptan-2-ol, 3,7-diméthyl-6-octèn-1-ol, 3,7-diméthyl-1-octanol, (2E)-3,7-diméthyl-2,6-octadièn-1-ol, cis-3,7-diméthyl-2,6-octadièn-1-ol, 3,7-diméthyl-octa-1,6-diène-3-ol, 2-(4-méthyl-1-cyclohex-3-ènyl)propan-2-ol, 4-méthyl-1-(1-méthyléthyl)-3-cyclohexèn-1-ol, (1R, 2S, 5R)-5-méthyl-2-(1-méthyléthyl)-cyclohexanol, (2E)-3,7-diméthyl-2,6-octadièn-1-ol et le 3-méthylbutan-1-ol. De manière préférée, des exemples d'alcools comprennent l'alpha,beta,2,2,3-pentaméthylcyclopent-3-ène-1-butanol, 3-(5,5,6-triméthylbicyclo[2.2.1]hept-2-yl) cyclohexan-1-ol (IBCH), cis-3-hexenol, méthyl-triméthylbicyclo-hexylméthyl-cyclopropyl méthanol, 3-méthylbutan-1-ol, linalol d'éthyle, tetrahydro linalol et le [1R-(1alpha,2beta,5alpha)]-5-méethyl-2-isopropylcyclohexanol (menthol).

Des exemples d'aldéhydes comprennent, mais ne sont pas limités à, les alcanals linéaires comprenant entre 8 et 18 atomes de carbones, 3,7-Dimethyl-2,6-octadienal (citral), citronellal, aldéhyde de Cyclamen, hydroxycitronellal, 3,7-Diméthyl-2,6-octadienal, Undécanal, alpha-méthyl-4-(1-méthyléthyl)-Benzènepropanal, 3-(4-isopropylphenyl)-2-methylpropanal, 2,4-diméthylcyclohex-3-ene-1-carbaldéhyde, 2,4-diméthylcyclohex-3-ene-1-carbaldéhyde, (2E)-2-Dodecenal, Octanal, Lauryl aldéhyde, Nonanal, (E)-2-Benzylideneoctanal, 2,4-diméthylcyclohex-3-ene-1-carbaldéhyde, 3-(4-éthylphényl)-2,2-diméthylpropanal, 4-Hydroxy-3-methoxybenzaldéhyde , 3-(4-tert-butylphényl)-2-methylpropanal, 3-(4-tert-butylphényl)propanal, 2,6,10-triméthylundec-9-ènal, 4(octahydro-4,7-méthano[5H]indèn-5-ylidène)butanal, 3-(3-propan-2-ylphényl)butanal, 7-hydroxy-3,7-diméthyloctanal (hydroxycitronellal, 3,7-diméthyl-7-hydroxy-octane-1-al), 4-(4-hydroxy-4-méthylpentyl)cyclohex-3-ène-1-carbaldehyde, octahydro-5-méthoxy-4,7-méthano-1H-indène-2-carboxaldehyde, aldéhyde d'alpha-méthyle cinnamique (2-méthyl-3-phényl-2-propènal), 4-méthoxybenzaldehyde (Aldéhyde anisique), aldéhyde en C10 (décanal), undéc-10-enal, aldéhyde en C12 (laurique ou dodécanal), acétaldéhyde de méthyl-nonyle (2-méthylundécanal), aldéhyde en C16, aldéhyde en C6 (hexanal), aldéhyde cinnamique (3-phényl-2-propénal), 3-éthyoxy-4-hydroxybenzaldéhyde (Ethylvanilline), aldéhyde d'hexyl cinnamique (2-benzylidèneheptanal), 3-phénylbutanal (3-phénylbutyraldehyde), 2,4-diméthylcyclohex-3-ène-1-carbaldehyde, 5-heptanal, 2,6-dimethylhept-5-enal, 4-hydroxy-3-méthoxybenzaldehyde (Vanilline), alpha-méthyl-1,3-benzodioxole-5-propionaldehyde, 4-isopropylbenzaldehyde, 3,7-dimethyl-6-octènal, 3,7-diméthyl-2,6-octadiènal, 4-(4-hydroxy-4-méthylpentyl)-3-cyclohexène-1-carboxaldehyde, trans-hex-2-ènal, 2,4,6-triméthyl-3-cyclohexène-1-carboxaldehyde, 2-(4-tert-butylbenzyl)propionaldehyde et le benzaldehyde. De manière préférée, des exemples d'aldéhydes comprennent le 2,4-diméthylcyclohex-3-ène-1-carbaldehyde, 5-heptanal, 2,6-diméthyl-hept-5-enal, 4-hydroxy-3-méthoxybenzaldehyde (Vanilline), alpha-méthyl-1,3-benzodioxole-5-propionaldehyde, citral et le benzaldehyde.

Des exemples de cétones comprennent, mais ne sont pas limités à, les ionones, isométhylionone, cédryle de méthyle, (E)-1-(2,6,6-triméthyl-1-cyclohex-2-enyl)but-2-en-1-one (alpha-damascone), 3-méthyl-2-[(2Z)-pent-2-en-1-yl]cyclopent-2-en-1-one (cis-jasmone), 4-(4-méthoxyphényl)-butan-2-one, 4(3)-(4-méthylpent-3-ènyl)cyclohex-3-ènecarbaldehyde, 1-(1,2,3,4,5,6,7,8-octahydro-2,3,8,8-tetraméthyl-2-naphthalenyl)éthanone, 3-Méthyl-4-(2,6,6-triméthyl-2-cyclohexényl)-3-butèn-2-one, (E)-4-(2,6,6-triméthyl-1-cyclohexen-1-yl)-3-buten-2-one, E)-4-(2,6,6-triméthylcyclohex-2-eneyl)- but-3-en-2-one, 7-Méthyl-2H-benzo-1,5-dioxepin-3(4H)-one, 1-(2,6,6-Triméthyl-1,3-cyclohexadienyl)-2-butèn-1-one, Méthyl hydroxypyrone, 2,2,5-Triméthyl-5-pentylcyclopentan-1-one, 1-(5,5-Diméthyl-1-cyclohexenyl)pent-4-en-1-one, 3-Méthyl-4-(2,6,6-triméthyl-2-cyclohexenyl)-3-buten-2-one, 2-[2-(4-Méthyl-3-cyclohexen-1-yl)propyl]-cyclopentanone, 1-(1,2,3,4,5,6,7,8-octahydro-2,3,8,8-tetraméthyl-2-naphthalenyl)éthanone, (E)-4-(2,6,6-triméthyl-1-cyclohexen-1-yl)-3-buten-2-one, 4-Phenylbutan-2-one

2-Cyclohexyl-1,6-heptadien-3-one, 1-(5,5-Diméthyl-1-cyclohexenyl)pent-4-en-1-one, 2-Buten-1-one, 1-(2,6,6-triméthyl-3-cyclohexen-1-yl)-, 2H-1-Benzopyran-2-one, 1-spiro(4.5)-7-decen-7-yl-4-penten-1-one and 1-spiro(4.5)-6-decen-7-yl-4-penten-1-one, (E)1-(2,6,6-Triméthyl-2-cyclohexen-1-yl)-2-buten-1-one, (E)-4-(2,6,6-triméthyl-1-cyclohexen-1-yl)-3-buten-2-one, Dihydro-5-pentyl-2(3H)-furanone, 2,2,5-Triméthyl-5-pentylcyclopentan-1-one, 1-(1,2,3,4,5,6,7,8-octahydro-2,3,8,8-tetraméthyl-2-naphthalenyl)éthanone, cétone de méthyl cédryl, 7-méthylbenzo[b][1,4]dioxepin-3-one, 1,7,7-triméthylbicyclo[2,2,1]heptan-2-one, 1-benzopyrane-2-one (Coumarine), 1-(2,6,6-triméthyl-1-cyclohex-3-ènyl)but-2-èn-1-one, butan-2,3-dione (Diacétyle), 1-(1,2,3,4,5,6,7,8-octahydro-2,3,8,8,-tetraméthyl-2-naphthyl)éthan-1-one, irones, 1-(2-naphthalényl)éthanone (2-acétonaphtone), menthone, carvone, 3-méthyl-2-pentyl-2-cyclopentènone, 1-(2,6,6-triméthyl-3-cyclohexen-1-yl)-2-butèn-1-one, 1-(2,6,6-triméthyl-2-cyclohexényl)hepta-1,6-dièn-3-one, 2-éthyl-3-hydroxy-4H-pyran-4-one, (5R)-2-méthyl-5-prop-1-èn-2-ylcyclohex-2-èn-1-one, 1-(6-tert-butyl-1,1-diméthyl-2,3-dihydro-1H-indèn-4-yl)éthanone, 1-(5,6,7,8-tetrahydro-3,5,5,6,8,8-hexaméthyl-2-naphthyl)éthan-1-one, 4-(2,6,6-triméthylcyclohex-2-ènyl)-but-3-ène-2-one, octan-2-one, 1-(1,2,3,4,5,6,7,8-Octahydro-2,3,8,8- Tétraméthyl-2-Napthyl)Ethan-1-one et la 1,3,4,6,7,8a-Hexahydro-1,1,5,5-tetramethyl-2H-2,4a-methanonaphthalen-8(5H)-one. De manière préférée, des exemples de cétones comprennent les irones, 1-(2-naphthalényl)éthanone (2-acétonaphtone), menthone, carvone, 3-méthyl-2-pentyl-2-cyclopentènone, 1-(1,2,3,4,5,6,7,8-Octahydro-2,3,8,8- Tétraméthyl-2-Napthyl)Ethan-1-one et la 1,3,4,6,7,8a-Hexahydro-1,1,5,5-tetramethyl-2H-2,4a-methanonaphthalen-8(5H)-one.

Des exemples de lactones comprennent, mais ne sont pas limités à la gamma décalactone (decan-4-olide), Décan-5-olide, Décan-4-olide, Undécan-4-olide gamma undécalactone (undecan-4-olide), cis-jasmone lactone, gamma undecalactone, delta octalacone, delta decalactone et la hexahydro-3,6-diméthyl-2(3H)-benzofuranone. De manière préférée, des exemples de lactones comprennent la gamma undecalactone, delta octalacone, delta decalactone et la hexahydro-3,6-diméthyl-2(3H)-benzofuranone.

Des exemples d'acétals comprennent, mais ne sont pas limités à, le 2,4- diméthyl tetrahydroindenodioxine, diacétal de l'aldéhyde phénylacétique, le glycérylacétal de phénylacetaldéhyde, le diéthyl acétal de citral, le diméthyl acétal de citral, 2,6-octadiènal, 1,1-diméthoxy-2-phényléthane et l'acide 3,7-diméthyl isomérisé. De manière préférée, des exemples d'acétals comprennent le glycérylacétal de phénylacetaldéhyde, le diéthyl acétal de citral, le diméthyl acétal de citral, 2,6-octadiènal et l'acide 3,7-diméthyl isomérisé.

Des exemples de nitriles comprennent, mais ne sont pas limités à, le 3,7-diméthyloct-6-ène nitrile (citronellyle nitrile), tridec-2-ènenitrile, 3-phényl-2-propènenitrile, 3,7-Diméthyl-6-ènenitrile, dodécanenitrile et le 3,7-diméthylnona-2,6-diènenitrile. De manière préférée, des exemples de nitriles comprennent le tridec-2-ènenitrile, 3-phényl-2-propènenitrile, dodécanenitrile et le 3,7-diméthylnona-2,6-diènenitrile.

Des exemples de phénols comprennent, mais ne sont pas limités à, l'eugénol (2-méthoxy-4-(2-propényl)-phénol), iso-eugénol, 5-méthyl-2-(1-méthyléthyl)-phénol, 2-éthoxy-4-méthylphénol, 2-isopropyl-5-methylphenol, 5-méthyl-2-(1-méthyléthyl)-Phénol, 2,6-di-tert-butyl-p-crésol et le 2-éthoxy-4-(méthoxyméthyl)-phénol.

Des exemples d'acides comprennent, mais ne sont pas limités à, l'acide pentanoïque, l'acide butyrique et l'acide 2-méthylpent-2-èn-1-oïque.

Des exemples de terpènes tels que des hydrocarbures terpéniques cycliques (par exemple sesquiterpéniques) ou non cycliques, comprennent, mais ne sont pas limités à, le limonène, 1-méthyl-4-isopropényl-1-cyclohexène, 1-méthyl-4-isopropyl-1,4-cyclohexadiène, 7-méthyl-3-méthylèneocta-1,6-diène, 1-méthyl-4-(1-méthyléthyl)-1,3-cyclohexadiène, 2,6,6-triméthylbicyclo[3.1.1]hept-2-ène, 6,6-diméthyl-2-méthylènebicyclo[3.1.1]heptane, 2,2-diméthyl-3-méthylènebicyclo-[2.2.1]-heptane, (3R-(3alpha,3abeta,6beta,7beta,8aalpha))-Octahydro-6-méthoxy-3,6,8,8-tetraméthyl-1H-3a,7-méthanoazulène, 4,11,11-triméthyl-8-méthylene-(1R,4E,9S)-Bicyclo[7.2.0.]undec-4-ene, [1R,(1R*,4E,9S*)]-4,11,11-triméthyl-8-méthylène-bicyclo[7.2.0]undec-4-ène, 1-méthyl-4-(1-méthyléthyl)benzène et les huiles essentielles à sesquiterpènes. De manière préférée, des exemples de terpènes comprennent des huiles essentielles à sesquiterpènes.

Des exemples de composés hétérocycliques azotés ou soufrés, saturés ou insaturés, comprennent, mais ne sont pas limités à, l'indole, 1,3-benzopyrrole, tétrahydro-4-méthyl-2-(2-méthyl-1-propényl)-2H-pyrane, 2-méthyl-pyrazine, 4-méthyl-5-hydroxyéthyl thiazole (2-(4-méthylthiazol-5-yl)éthanol), 6-tert-butylquinoline, 6-(isopropyl)quinoline, (3aR-(3aalpha,5abeta,9aalpha,9bbeta))-Dodécahydro-3a,6,6,9a-tetraméthylnaphtho(2,1-b)furane, 2-Isopropyl-4-méthyl-1,3-thiazole, cis-2-méthyl-4-propyl-1,3-oxathiane, 6(8)-(1-Methylpropyl)Quinoléine et les pyrazines. De manière préférée, des exemples de composés hétérocycliques azotés ou soufrés, saturés ou insaturés, comprennent la 6-tert-butylquinoline, 6-(isopropyl)quinoline, cis-2-méthyl-4-propyl-1,3-oxathiane, 6(8)-(1-Methylpropyl)Quinoléine et les pyrazines.

Des exemples de produits complexes d'origine naturelle comprennent, mais ne sont pas limités à, des huiles essentielles extraites à partir des différentes parties de plantes (fleurs, tiges, feuilles, fruits, écorces, racines, parties boisées, herbes, aiguilles, sève et gommes), des résinoïdes, des concrètes, ou absolues obtenues à partir de ces derniers. De manière préférée, des exemples de produits complexes d'origine naturelle comprennent de l'huile d'armoise herbe blanche (Artemisia Herba-Alba), Huile de feuille de Pogostemon Cablin (Pogostemon Cablin Leaf Oil), Huile d'écorce de Citrus nobilis (Citrus nobilis peel oil), Huile de feuille de Barosma Betulina (Barosma Betulina Leaf Extract), extrait de l'écorce de citronnier (Citrus Limon Peel extract), de l'huile de feuille d'Eucalyptus globulus, huile de Dipterocarpus turbinatus (Dipterocarpus turbinatus Balsam Oil), huile de feuille de Pogostemon cablin, huile de feuille de Rosmarinus officinalis, huile de Juniperus virginiana, huile de feuille de menthe des champs (Mentha Arvensis), huile de feuille de menthe verte (Mentha viridis), huile d'écorce de Citrus Aurantium Dulcis, extrait d'écorce de Citrus Aurantium Dulcis, huile de feuille de Cyprès méditerranéen (Cupressus sempervirens), huile de feuille de patchouli (Pogostemon cablin), huile de Cèdre Texas (Juniperus Mexicana) et l'huile de Lavandula Hybride (Lavandula Hybrida).

Le parfum selon l'invention peut comprendre des ingrédients d'origine naturelle ou synthétique. Le choix de ce parfum dépend d'une part de l'effet odorant recherché et d'autre part de la nature du produit qui la contient.

Selon une possibilité, le complexe parfumant et la composition selon l'invention comprend des parfums contenant moins de 18 % en poids d'alcool ramifié avec au minimum 8 atomes de carbone par rapport au poids total dudit parfum.

Le parfum peut être utilisé dans des proportions qui varient en fonction de l'usage souhaité.

Le parfum peut représenter 5 à 40 %, 7 à 30 % ou encore 10 à 20 % en poids par rapport au poids total de ladite composition.

Le C12-13 pareth-9 est un tensioactif non-ionique avantageux compte tenu de son coût faible et de sa biodégradabilité en aérobiose. Par ailleurs, le C12-13 pareth-9 est non-irritant pour la peau et ne présente pas d'odeur. En particulier, la biodégradabilité du C12-13 pareth-9 est supérieure à 60 % selon la norme OCBE 301B.

Le C12-13 pareth-9 peut être utilisé dans des proportions qui varient en fonction de l'usage souhaité.

De manière préférée, le C12-13 pareth-9 représente 5 à 30 %, préférentiellement 7 à 25 % et encore plus préférentiellement 9 à 20 % en poids par rapport au poids total de ladite composition.

L'isopentyldiol (appelé également isoprène glycol ou 3-méthyl 1,3-butanediol) est un co-solvant hydrophile qui permet de diminuer la quantité de tensioactifs à ajouter afin d'obtenir une composition stable. En outre, ce co-solvant permet l'obtention de compositions translucides, ne dénature pas les parfums, n'est ni collant, ni irritant et n'est pas sensible à l'oxydation par l'air. Il permet également d'obtenir un toucher sec à l'étalement au bout de quelques minutes, préférentiellement au bout d'une minute et encore plus préférentiellement au bout de quelques secondes.

L'isopentyldiol n'est pas un diol vicinal.

« Par diol vicinal » on entend un alcool comprenant au moins deux groupes hydroxyles, deux des groupes hydroxyles étant attachés à des atomes de carbone adjacents.

L'isopentyldiol peut être utilisé dans des proportions qui varient en fonction de l'usage souhaité.

De manière préférée, l'isopentyldiol représente 5 à 30 %, préférentiellement 7 à 25 % et encore plus préférentiellement 10 à 20 % en poids par rapport au poids total de ladite composition.

Selon un mode de réalisation particulier, la composition selon l'invention comprend en outre au moins un additif choisi parmi les agents anti-mousses, agents antioxydants, agents chélatants, filtres UV, conservateurs, agents épaississants, ingrédients actifs cosmétiques, agents hydratants, humectants, adoucissants, pigments, colorants, agent réfrigérant, agents ajusteurs de pH, agents bactéricides, agents bactériostatiques, insecticides, agents répulsifs et leurs mélanges.

Les additifs sont présents en une quantité allant de 0 à 5 %, de préférence de 0,001 à 2 %, plus préférentiellement de 0,05 à 1 %, et encore plus préférentiellement de 0,001 à 0,1 % en poids par rapport au poids total de ladite composition.

De manière préférée, la composition selon la présente invention comprend :
- 5,00 % à 40,00 %, préférentiellement 10,00 % à 30,00 %, encore plus préférentiellement 20,00 % en poids de parfum par rapport au poids total de ladite composition,
- 5,00 % à 30,00 %, préférentiellement 10,00 % à 25,00 %, encore plus préférentiellement 20,00 % en poids de C12-13 Pareth-9 par rapport au poids total de ladite composition, et
- 5,00 % à 30,00 %, préférentiellement 10,00 % à 25,00 %, encore plus préférentiellement 20,00 % en poids d'isopentyldiol par rapport au poids total de ladite composition, et

De manière préférée, la composition selon la présente invention comprend :
- 5,00 % à 40,00 %, préférentiellement 6,00 % à 30,00 %, encore plus préférentiellement 7,00 % en poids de parfum par rapport au poids total de ladite composition,
- 5,00 % à 30,00 %, préférentiellement 7,00 % à 20,00 %, encore plus préférentiellement 8,00 % en poids de C12-13 Pareth-9 par rapport au poids total de ladite composition, et
- 5,00 % à 30,00 %, préférentiellement 6,00 % à 20,00 %, encore plus préférentiellement 7,00 % en poids d'isopentyldiol par rapport au poids total de ladite composition, et

De manière préférée, la composition selon la présente invention comprend :
- 5,00 % à 40,00 %, préférentiellement 5,00 % à 30,00 %, encore plus préférentiellement 5,00 % en poids de parfum par rapport au poids total de ladite composition,
- 5,00 % à 30,00 %, préférentiellement 7,00 % à 20,00 %, encore plus préférentiellement 9,00 % en poids de C12-13 Pareth-9 par rapport au poids total de ladite composition, et
- 5,00 % à 30,00 %, préférentiellement 10,00 % à 20,00 %, encore plus préférentiellement 15,00 % en poids d'isopentyldiol par rapport au poids total de ladite composition, et

De manière préférée, la composition selon la présente invention comprend :
- 5,00 % à 40,00 %, préférentiellement 10,00 % à 30,00 %, encore plus préférentiellement 12,00 % en poids de parfum par rapport au poids total de ladite composition,
- 5,00 % à 30,00 %, préférentiellement 10,00 % à 20,00 %, encore plus préférentiellement 13,00 % en poids de C12-13 Pareth-9 par rapport au poids total de ladite composition, et
- 5,00 % à 30,00 %, préférentiellement 10,00 % à 20,00 %, encore plus préférentiellement 15,00 % en poids d'isopentyldiol par rapport au poids total de ladite composition.

De manière préférée, la composition selon la présente invention comprend :
- 5,00 % à 40,00 %, préférentiellement 10,00 % à 30,00 %, encore plus préférentiellement 15,00 % en poids de parfum par rapport au poids total de ladite composition,
- 5,00 % à 30,00 %, préférentiellement 10,00 % à 20,00 %, encore plus préférentiellement 15,00 % en poids de C12-13 Pareth-9 par rapport au poids total de ladite composition, et
- 5,00 % à 30,00 %, préférentiellement 10,00 % à 20,00 %, encore plus préférentiellement 15,00 % en poids d'isopentyldiol par rapport au poids total de ladite composition.

De manière préférée, la composition selon la présente invention comprend :
- 5,00 % à 40,00 %, préférentiellement 10,00 % à 40,00 %, encore plus préférentiellement 40,00 % en poids de parfum par rapport au poids total de ladite composition,
- 5,00 % à 30,00 %, préférentiellement 15,00 % à 25,00 %, encore plus préférentiellement 25,00 % en poids de C12-13 Pareth-9 par rapport au poids total de ladite composition, et
- 5,00 % à 30,00 %, préférentiellement 10,00 % à 20,00 %, encore plus préférentiellement 15,00 % en poids d'isopentyldiol par rapport au poids total de ladite composition.

De manière préférée, la composition selon la présente invention comprend :
- 5,00 % à 40,00 %, préférentiellement 10,00 % à 40,00 %, encore plus préférentiellement 10,00 % en poids de parfum par rapport au poids total de ladite composition,
- 5,00 % à 30,00 %, préférentiellement 15,00 % à 25,00 %, encore plus préférentiellement 16,00 % en poids d'isopentyldiol par rapport au poids total de ladite composition, et
- 5,00 % à 30,00 %, préférentiellement 10,00 % à 20,00 %, encore plus préférentiellement 9,00 % en poids de C12-13 Pareth-9 par rapport au poids total de ladite composition.

Tous les pourcentages sont donnés par rapport au poids total de ladite composition.

De manière préférée, la composition est une composition aqueuse.

De manière préférée, la composition selon l'invention est une microémulsion.

Par « microémulsion », on entend une émulsion dont les micelles ont une taille comprise entre 100 et 200 nm, et donc invisibles à l'œil nu, donnant ainsi un aspect transparent.

De manière préférée, la composition selon l'invention est exempte d'éthanol.

La composition selon l'invention comprend une teneur très faible en Composé Organique Volatile (COV), notamment inférieure ou égale 400 à g/L.

Selon un mode de réalisation particulier, la composition est exempte de Composé Organique Volatile (COV).

La composition selon l'invention ne contient pas de co-solvant choisi dans le groupe comprenant l'isosorbide, le solketal et des éthers de ceux-ci.

De manière préférée, la composition selon l'invention ne contient pas de sulfates.

De manière préférée, la composition selon l'invention ne contient pas de diol vicinal.

La composition selon l'invention est destinée à être appliquée ou vaporisée sur une surface ou dans l'air afin de conférer une odeur agréable.

Par « odeur agréable », on entend une odeur qui est détectée par le sens olfactif de l'être humain et qui est perçue comme agréable.

La présente invention a également pour objet une base cosmétique comprenant la composition selon la présente invention.

Par « base cosmétique », on entend un produit cosmétique ou un produit de soin d'hygiène sous forme d'une crème, d'une émulsion, d'une mousse, d'une cire, d'une huile, d'une lotion, d'un gel, d'une suspension, d'une brume, d'une solution, d'une poudre, d'un baume, d'un sérum, d'un masque ou d'un gommage. Des exemples de produits cosmétiques, comprennent, mais ne sont pas limités à, des parfums, eaux de parfums, eaux de toilette pour adultes et bébés, produits capillaires, produits de rasage et d'après-rasage, huiles essentielles, soins pour la peau, déodorants, anti-transpirants, produits dépilatoires, produits auto-bronzants, protections solaires, produits de maquillage, brume pour le corps etc. Des exemples de soins d'hygiène comprennent, mais ne sont pas limités à, des lingettes rafraîchissantes et/ou nettoyantes, talcs, couches, bavoirs, mouchoirs, serviettes en papiers, savons, gels douches, shampooings et autres produits de nettoyage corporel, soins bucco-dentaires, produits d'hygiène féminine, désodorisants, déodorants, etc.

La présente invention a également pour objet une base détergente comprenant la composition selon la présente invention.

Par « base détergente », on entend des produits d'entretien ou de nettoyage. Des exemples de produits d'entretien ou de nettoyage comprennent, mais ne sont pas limités à, des détergents pour surfaces, pour textiles et pour vaisselle, des agents de blanchiment, agents adoucissants, agents assouplissants, parfums pour le lave-linge ou le séchoir à linge, produits décapants, vernis et autres produits ménagers.

La présente invention a également pour objet des produits désherbants et engrais comprenant la composition selon la présente invention.

La présente invention a également pour objet un parfum d'ambiance comprenant la composition selon la présente invention.

Par « parfum d'ambiance », on entend des produits destinés à parfumer l'air. Les produits d'ambiance peuvent, de façon non limitative, être contenus dans des aérosols, vaporisateurs, sprays, bougies, gels parfumés, supports solides, brûles parfums, diffuseurs à bâtonnets, encens, billes et diffuseurs de parfums. Le parfum d'ambiance de la présente invention peut être utilisé pour les espaces clos publics, professionnels ou privés; par exemple, la voiture, la maison, les bâtiments administratifs, les transports en commun, les musées, les monuments historiques, les églises, les caves, les écoles, les restaurants, les cinémas, les hôpitaux, les usines, les stations de traitement d'eau ou de déchets, les boutiques et les hôtels. Le parfum d'ambiance peut également être utilisé dans les systèmes à air conditionné.

La présente invention concerne également des matériaux comprenant ou recouverts par la composition selon l'invention, notamment des polymères comme par exemple des plastiques, des macromolécules comme par exemple la cellulose, etc. Par exemple, la composition selon l'invention pourra être utilisée comme parfum pour le linge notamment comme brume d'oreiller.

La composition selon l'invention est destinée à être appliquée sous forme concentrée ou non dans des bases cosmétiques, bases détergentes ou parfums d'ambiance.

La présente invention a également pour objet l'utilisation de la composition selon l'invention pour préparer une composition cosmétique, une base détergente ou un parfum d'ambiance.

La présente invention a également pour objet l'utilisation de la composition selon l'invention, de la base cosmétique, de la base détergente ou du parfum d'ambiance pour masquer les mauvaises odeurs.

Par « mauvaises odeurs », on entend des odeurs qui sont détectées par le sens olfactif de l'être humain et qui sont perçues comme offensantes ou désagréables. Au sens de la présente invention, les mauvaises odeurs à masquer peuvent résulter de manière intrinsèque de la base cosmétique, détergente ou du parfum d'ambiance luimême, ou bien provenir de l'environnement extérieur.

Par « masquer les mauvaises odeurs » on entend la modification de la perception qualitative de l'odeur via l'ajout d'une substance ou d'un mélange de substance capable de fournir une odeur nouvelle, distincte et agréable.

Dans un mode de réalisation particulier, la composition selon la présente invention masque les mauvaises odeurs sans pour autant dégrader les molécules responsables des mauvaises odeurs. En effet, les molécules de la composition masquent olfactivement les molécules responsables des mauvaises odeurs.

La composition selon la présente invention peut être préparée par simple mélange des ingrédients.

Les modes de réalisation détaillés ci-dessus peuvent être aisément combinés les uns avec les autres de façon non limitative.

La présente invention sera décrite plus en détail à l'aide des exemples suivants qui ont un caractère purement illustratif.

### EXEMPLES

### Exemple 1 : Composition parfumée No. 1

Les composants de la composition parfumée No. 1, décrits dans le tableau 1, sont mélangés afin d'obtenir la composition parfumée No. 1.

**Tableau 1**

| Désignation du produit | % En poids Quantité |
|---|---|
| Parfum 1 | 20,000 % |
| C12-13 Pareth-9 | 20,000 % |
| Isopentyldiol | 20,000 % |
| Eau distillée | 39,250 % |
| Phénoxyéthanol, Chlorphénésine, Caprylyl Glycol | 0,750 % |
| Siméthicone | 1 × 10-3 % |

Les composants du parfum 1, décrits dans le tableau 2, sont mélangés afin d'obtenir le parfum 1.

**Tableau 2**

| Désignation du produit | % En poids Quantité |
|---|---|
| Triéthyle citrate | 7,000 % |
| 4-tert-Butylcyclohexyl Acétate | 10,000 % |
| 6-tert-Butylquinoline | 0,300 % |
| (3R-(3alpha,3abeta,6beta,7beta,8aalpha))-Octahydro-6-méthoxy-3,6,8,8-tetraméthyl-1H-3a,7-méthanoazulène | 5,000 % |
| (3R-(3alpha,3abeta,6alpha,7beta,8aalpha))-Octahydro-3,6,8,8-tetraméthyl-1H-3a,7-méthanoazulèen-5-yl Acétate | 40,000 % |
| 1-(1,2,3,4,5,6,7,8-octahydro-2,3,8,8-tetraméthyl-2-naphthalenyl)éthanone | 30,000 % |
| Suederal LT 2010-2015^{®}, société IFF | 0,500 % |
| 3-Méthyl-4-(2,6,6-triméthyl-2-cyclohexényl)-3-butèn-2-one | 5,000 % |
| 3,7-Diméthyl-2,6-octadienal | 0,500 % |
| 3,7-Diméthyl octa-1,6-diene-3-yl acétate | 1,700 % |

### Exemple 2 : Composition parfumée No. 2

Les composants de la composition parfumée No. 2, décrits dans le tableau 3, sont mélangés afin d'obtenir la composition parfumée No. 2.

**Tableau 3**

| Désignation du produit | % En poids Quantité |
|---|---|
| Parfum 1 | 7,000 % |
| C12-13 Pareth-9 | 8,000 % |
| Isopentyldiol | 7,000 % |
| Eau distillée | 78,000 % |

### Exemple 3 : Composition parfumée No. 3

Les composants de la composition parfumée No. 3, décrits dans le tableau 4, sont mélangés afin d'obtenir la composition parfumée No. 3.

**Tableau 4**

| Désignation du produit | % En poids Quantité |
|---|---|
| Parfum 2 | 5,000 % |
| C12-13 Pareth-9 | 9,000 % |
| Isopentyldiol | 15,000 % |
| Eau distillée | 70,250 % |
| 2-phénoxyéthanol (et) Octane-1,2-diol (et) 3-(4-Chlorophénoxy)-1,2-propanediol | 0,750 % |

Les composants du parfum 2, décrits dans le tableau 5, sont mélangés afin d'obtenir le parfum 2.

**Tableau 5**

| Désignation du produit | % En poids |
|---|---|
| Isopentyl acétate | 0,017 |
| Méthyl Phénylacétate | 0,500 |
| (Z)-Hex-3-enyl acétate | 0,125 |
| 3,7-Diméthyl octa-1,6-diene-3-yl acétate | 2,500 |
| 3-Méthyl-2-butényl acétate | 0,017 |
| Benzèneméthanol, a-Methyl-, Acétate | 0,125 |
| Undécanal | 0,008 |
| Undécan-4-olide | 0,017 |
| Benzènepropanal, .alpha.-méthyl-4-(1-méthyléthyl)- | 0,008 |
| (3aR-(3aalpha,5abeta,9aalpha,9bbeta))-Dodécahydro-3a,6,6,9a-tetraméthylnaphtho(2,1-b)furane | 0,042 |
| Méthyl 2-aminobenzoate | 0,042 |
| 2-Phényléthanol | 0,833 |
| cis-Hex-3-en-1-ol | 0,125 |
| 4-Méthyl-3-decen-5-ol | 0,083 |
| 7-Méthyl-2H-benzo-1,5-dioxepin-3(4H)-one | 0,042 |
| 2-Propényl (cyclohexyloxy)acétate | 0,042 |
| Decan-5-olide | 0,008 |
| 1-(2,6,6-Triméthyl-1,3-cyclohexadienyl)-2-butèn-1-one | 0,025 |
| 2,6-diméthyloct-7-en-2-ol | 0,017 |
| 1,1'-Oxydipropan-2-ol; | 52,764 |
| 3,7-Diméthyl-1,6-nonadien-3-ol | 1,667 |
| Huile de citron (Citrus Limon Fruit Oil) | 0,833 |
| Méthyl hydroxypyrone | 0,001 |
| 2,2,5-Triméthyl-5-pentylcyclopentan-1 -one | 0,027 |
| 3,4,4a,5,8,8a-Hexahydro-3',7'-diméthylspiro(1,4-methanonaphthalene-2(1H),2'-oxirane) | 0,002 |
| cis-2-Méthyl-4-propyl-1,3-oxathiane | 0,002 |
| Méthyl 2,4-dihydroxy-3,6-diméthylbenzoate | 0,033 |
| 2,4-diméthylcyclohex-3-ene-1-carbaldehyde | 0,052 |
| méthyl 3-oxo-2-pentylcyclopentaneacétate | 20,018 |
| Triéthyl citrate | 0,080 |
| 1-(5,5-Diméthyl-1-cyclohexenyl)pent-4-en-1-one | 0,001 |
| 3,7-Diméthyl-6-octen-1-ol | 0,005 |
| 6,6-Diméthyl-2-méthylènebicyclo[3.1.1]heptane | 0,013 |
| 2,6,6-Triméthylbicyclo[3.1.1]hept-2-ène | 0,001 |
| Huile de feuille de Pogostemon Cablin (Pogostemon Cablin Leaf Oil) | 0,042 |
| Tétrahydro-4-méthyl-2-(2-méthylpropyl)-2H-pyran-4-ol | 5,417 |
| 1,4-Dioxacycloheptadecane-5,17-dione | 6,250 |
| Décan-4-olide | 0,042 |
| alpha-Méthyl-1,3-benzodioxole-5-propionaldehyde | 1,250 |
| (E)-4-(2,6,6-triméthyl-1-cyclohexen-1-yl)-3-buten-2-one | 1,667 |
| 2-éthoxy-4-(méthoxyméthyl)-Phénol | 0,017 |
| 3,7-Diméthyl octa-1,6-diene-3-ol | 2,083 |
| 1,4-Dioxacyclohexadecane-5,16-Dione | 0,017 |
| 3-Méthyl-5-cyclopentadecen-1-one | 0,083 |
| benzyl 2-hydroxybenzoate | 0,583 |
| (Z)-3-Hexenyl 2-hydroxybenzoate | 0,583 |
| p-menth-1-en-8-ol | 0,008 |
| 2-(1,1-Diméthylethyl)Cyclohexyl Acétate | 0,025 |
| Huile d'écorce de Citrus nobilis (Citrus nobilis peel oil) | 0,833 |
| Huile d'écorce de Citrus Aurantium Dulcis (Citrus Aurantium Dulcis (Orange) Peel Oil) | 0,833 |
| 2,4,6-Triméthyl-4-phenyl-1,3-dioxane | 0,025 |
| 4-(2,6,6-Triméthylcyclohex-2-eneyl)-but-3-ene-2-one | 0,167 |

### Exemple 4 : Composition parfumée No. 4

Les composants de la composition parfumée No. 4, décrits dans le tableau 6, sont mélangés afin d'obtenir la composition parfumée No. 4.

**Tableau 6**

| Désignation du produit | % En poids Quantité |
|---|---|
| Parfum 3 | 12,000 % |
| C12-13 Pareth-9 | 13,000 % |
| Isopentyldiol | 15,000 % |
| Eau distillée | 59,250 % |
| 2-phénoxyéthanol (et) Octane-1,2-diol (et) 3-(4-Chlorophénoxy)-1,2-propanediol | 0,750 % |

Les composants du parfum 3, décrits dans le tableau 7, sont mélangés afin d'obtenir le parfum 3.

**Tableau 7**

| Désignation du produit | % En poids |
|---|---|
| 1,1'-Oxydipropan-2-ol | 30,47 % |
| 2,6-diméthyloct-7-en-2-ol | 10,77 % |
| 3,7-Diméthyl octa-1,6-diene-3-ol | 9,23 % |
| méthyl 3-oxo-2-pentylcyclopentaneacétate | 6,15 % |
| 2-(1,1-Diméthyléthyl)Cyclohexyl Acétate | 4,62 % |
| 2-Phényléthanol | 3,85 % |
| 2-éthyl-4-(2,2,3-triméthyl-3-cyclopenten-1-yl)-2-buten-1-ol | 3,08 % |
| Acide benzoïque, 2-hydroxy-, 2-hexyl ester | 3,08 % |
| 3a,4,5,6,7,7a-Hexahydro-4,7-méthanoinden-6-yl Acétate | 3,08 % |
| phénylméthanol | 2,31 % |
| 4-Méthyl-3-decen-5-ol | 2,15 % |
| 3-Méthyl-4-(2,6,6-triméthyl-2-cyclohexenyl)-3-buten-2-one | 1,69 % |
| 2-[2-(4-Méthyl-3-cyclohexen-1-yl)propyl]-cyclopentanone | 1,54 % |
| 3a,4,5,6,7,7a-Hexahydro-4,7-methano-1H-inden-6-yl Propanoate | 1,54 % |
| Extrait de fleur de Citrus Aurantium Dulcis (Citrus Aurantium Dulcis Flower Extract) | 1,54 % |
| 1-(1,2,3,4,5,6,7,8-octahydro-2,3,8,8-tetraméthyl-2-naphthalenyl)éthanone | 1,54 % |
| 2,6-Diméthyloctan-2-ol | 1,23 % |
| Benzène, 1,1'-Oxybis | 1,08 % |
| 2-Isopropyl-4-méthyl-1,3-thiazole | 0,01 % |
| Triéthyl citrate | 1,07 % |
| 3,7-Diméthyl-6-octen-1-ol | 1,08 % |
| 2,4-diméthylcyclohex-3-ene-1-carbaldehyde | 0,92 % |
| (E)-4-(2,6,6-triméthyl-1-cyclohexen-1-yl)-3-buten-2-one | 0,77 % |
| Methyl 2-naphthyl ether | 0,77 % |
| Undecan-4-olide | 0,62 % |
| 4-Phenylbutan-2-one | 0,62 % |
| 2-Cyclohexyl-1,6-heptadien-3-one | 0,46 % |
| Cyclohexanol, 4-(1,1-diméthyléthyl)- | 0,46 % |
| éthyl 2-méthylbutyrate | 0,46 % |
| 3a,4,5,6,7,7a-Hexahydro-4,7-méthano-1H-inden-5-yl 2-méthylpropanoate | 0,46 % |
| 2-Méthy I-1 -phénylpropan-2-ol | 0,46 % |
| alpha,alpha-Diméthylphénéthyl Acétate | 0,46 % |
| 2,6-Octadien-1-ol, 3,7-diméthyl-, (2E)- | 0,31 % |
| 3,7-Diméthyl octa-1 ,6-diene-3-yl acétate | 0,31 % |
| Hexan-1-ol | 0,15 % |
| Bicyclo[2.2.1]heptan-2-ol,1 ,7,7-triméthyl-, acétate, exo- | 0,15 % |
| Décan-4-olide | 0,15 % |
| 1-(5,5-Diméthyl-1-cyclohexenyl)pent-4-en-1-one | 0,15 % |
| exo-1,7,7-Triméthylbicyclo[2.2.1]heptan-2-ol | 0,15 % |
| 2-Buten-1-one, 1-(2,6,6-triméthyl-3-cyclohexen-1-yl)- | 0,15 % |
| éthyl hexanoate | 0,15 % |
| 2H-1-Benzopyran-2-one | 0,15 % |
| 2-(2,2,7,7-tetraméthyltricyclo[6.2.1.0 (1,6)]undec-5(4)-en-5-yl)propan-1-ol | 0,15 % |
| 3,7-Diméthyloct-6-enenitrile | 0,15 % |
| Benzaldehyde | 0,09 % |
| 1-spiro(4.5)-7-decen-7-yl-4-penten-1-one and 1-spiro(4.5)-6-decen-7-yl-4-penten-1-one | 0,08 % |
| 1-Phenyléthanol | 0,08 % |
| éthyl 2-naphthyl éther | 0,06 % |

### Exemple 5 : Composition parfumée No. 5

Les composants de la composition parfumée No. 5, décrits dans le tableau 8, sont mélangés afin d'obtenir la composition parfumée No. 5.

**Tableau 8**

| Désignation du produit | % En poids Quantité |
|---|---|
| Parfum 4 | 15,000 % |
| C12-13 Pareth-9 | 15,000 % |
| Isopentyldiol | 15,000 % |
| Eau distillée | 54,250 % |
| 2-phenoxyethanol (et) Octane-1,2-diol (et) 3-(4-Chlorophenoxy)-1,2-propanediol | 0,750 % |

Les composants du parfum 4, décrits dans le tableau 9, sont mélangés afin d'obtenir le parfum 4.

**Tableau 9**

| Désignation du produit | % En poids |
|---|---|
| 1,1'-Oxydipropan-2-ol | 60,620 % |
| 3,7-Diméthyloctan-3-ol | 6,897 % |
| 3,7-Diméthyl octa-1 ,6-diene-3-yl acétate | 5,172 % |
| 3,7-Diméthyloct-6-enenitrile | 2,069 % |
| cis-3,7-Diméthyl-2,6-octadien-1-ol | 2,414 % |
| méthyl 3-oxo-2-pentylcyclopentaneacétate | 3,793 % |
| (2E)-2-Dodecenal | 0,161 % |
| 3,7-Diméthyl-6-octen-1-ol | 3,448 % |
| (E)1-(2,6,6-Triméthyl-2-cyclohexen-1-yl)-2-buten-1-one | 0,276 % |
| (E)-4-(2,6,6-triméthyl-1-cyclohexen-1-yl)-3-buten-2-one | 2,207 % |
| Décanal | 2,414 % |
| cis-Hex-3-en-1-ol; | 0,621 % |
| 3,7-Diméthyl-2,6-octadienal | 2,069 % |
| Méthyl 2-(méthylamino)benzoate | 0,483 % |
| Hex-2-en-1-ol | 0,138 % |
| GALBEX 183 EB^{®}, société Firmenich | 0,011 % |
| Undeca-1,3,5-triene | 0,002 % |
| Phénol, 5-méthyl-2-(1-méthyléthyl)- | 0,065 % |
| 1-Méthyl-4-(1-méthyléthyl)benzène | 0,214 % |
| cis-2-Méthyl-4-propyl-1,3-oxathiane | 0,035 % |
| 3,7-Diméthyl octa-1 ,6-diène-3-ol | 0,081 % |
| Octanal | 0,139 % |
| Lauryl aldéhyde | 0,001 % |
| 2-Méthyl-4-phénylbutan-2-ol | 0,621 % |
| Nonanal | 0,345 % |
| Huile d'écorce de Citrus Aurantium Dulcis (Citrus Aurantium Dulcis (Orange) Peel Oil) | 0,552 % |
| Dihydro-5-pentyl-2(3H)-furanone | 0,069 % |
| 2,4-Diméthyl-4-phényltetrahydrofuran | 0,024 % |
| 10-Undécenoic Acid, éthyl Ester | 0,024 % |
| éthyl 2-méthylpentanoate | 0,021 % |
| 2,6-Octadien-1-ol, 3,7-diméthyl-, (2E)- | 0,276 % |
| cis-3,7-Diméthyl-2,6-octadienyl éthanoate | 0,552 % |
| 2,2,5-Triméthyl-5-pentylcyclopentan-1-one | 0,276 % |
| p-menth-1-en-8-ol | 0,041 % |
| 1-(1,2,3,4,5,6,7,8-octahydro-2,3,8,8-tetraméthyl-2-naphthalenyl)éthanone | 1,034 % |
| (E)-2-Benzylideneoctanal | 0,552 % |
| 2,4-diméthylcyclohex-3-ene-1-carbaldehyde | 0,552 % |
| 1,4-Dioxacycloheptadecane-5,17-dione | 0,414 % |
| Huile de feuille de Barosma Betulina (Barosma Betulina Leaf Extract ) | 0,345 % |
| Bicyclo[7.2.0.]undec-4-ene, 4,11,11-triméthyl-8-méthylene-, (1R,4E,9S) | 0,014 % |
| 3-(4-éthylphényl)-2,2-diméthylpropanal | 0,276 % |
| benzyl 2-hydroxybenzoate | 0,069 % |
| (Z)-Hex-3-enyl acétate | 0,345 % |
| 2,6-diméthyloct-7-en-2-ol | 0,041 % |
| 4-Hydroxy-3-methoxybenzaldéhyde | 0,069 % |
| 2H-1-Benzopyran-2-one | 0,034 % |
| Decan-4-olide | 0,041 % |
| Acide benzoïque, 2-hydroxy-, 2-hexyl ester | 0,028 % |
| 2-Propényl (cyclohexyloxy)acétate | 0,048 % |
| Acide acétique, (3-Methylbutoxy), 2-Propényl Ester | 0,007 % |

### Exemple 6 : Composition parfumée No. 6

Les composants de la composition parfumée No. 6, décrits dans le tableau 10, sont mélangés afin d'obtenir la composition parfumée No. 6.

**Tableau 10**

| Désignation du produit | % En poids Quantité |
|---|---|
| Parfum 5 | 40,000 % |
| C12-13 Pareth-9 | 25,000 % |
| Isopentyldiol | 15,000 % |
| Eau distillée | 19,250 % |
| 2-phenoxyethanol (et) Octane-1,2-diol (et) 3-(4-Chlorophenoxy)-1,2-propanediol | 0,750 % |

Les composants du parfum 5, décrits dans le tableau 11, sont mélangés afin d'obtenir le parfum 5.

**Tableau 11**

| Désignation du produit | % En poids |
|---|---|
| 2-Phényléthanol | 40,00 % |
| Phénéthyl acétate | 10,00 % |
| 3-Phénylpropan-1-ol | 15,00 % |
| Acétate d'hexyle | 5,00 % |
| (1R, 2S, 5R)-5-Méthyl-2-(1-méthyléthyl)-cyclohexanol éthanoate | 10,00 % |
| phénylméthanol | 20,00 % |

### Exemple 7 : Complexe parfumant No. 1

Les composants décrits dans le tableau 12, sont mélangés afin d'obtenir le complexe parfumant No. 1.

**Tableau 12**

| Désignation du produit | % En poids Quantité |
|---|---|
| Parfum 2 | 28,000 % |
| C12-13 Pareth-9 | 26,000 % |
| Isopentydiol | 46,000 % |

### Exemple 8 : Composition parfumante No. 7

Les composants décrits dans le tableau 13, sont mélangés afin d'obtenir la composition parfumante No. 7.

**Tableau 13**

| Désignation du produit | % En poids Quantité |
|---|---|
| Complexe parfumant No. 1 | 35,000 % |
| Eau distillée | 64,250 % |
| 2-phénoxyéthanol (et) Octane-1,2-diol (et 3-(4-Chlorophénoxy)-1 ,2-propanediol | 0,750 % |

### Exemple 9 : Complexe parfumant No. 2

Les composants décrits dans le tableau 14, sont mélangés afin d'obtenir le complexe parfumant No. 2.

**Tableau 14**

| Désignation du produit | % En poids Quantité |
|---|---|
| Parfum 2 | 23,300 % |
| C12-13 Pareth-9 | 26,700 % |
| Isopentydiol | 50,000 % |

### Exemple 10 : Composition parfumante No. 8

Les composants décrits dans le tableau 15, sont mélangés afin d'obtenir la composition parfumante No. 8.

**Tableau 15**

| Désignation du produit | % En poids Quantité |
|---|---|
| Complexe parfumant No. 2 | 35,000 % |
| Eau distillée | 64,250 % |
| 2-phénoxyéthanol (et) Octane-1,2-diol (et 3-(4-Chlorophénoxy)-1 ,2-propanediol | 0,750 % |

Les compositions parfumées N°7 et N°8 sont transparentes, peu collantes et puissantes olfactivement.

### Exemple comparatif 1 : Composition parfumée comparative A

Les composants de la composition parfumée comparative A, décrits dans le tableau 16, sont mélangés afin d'obtenir la composition parfumée comparative A.

**Tableau 16**

| Désignation du produit | % En poids Quantité |
|---|---|
| Mélange constitué de citronellol, dihydro myrcénol, éthyl linalol, et linalool | 7,000 % |
| Mélange de tensioactif A | 8,000 % |
| Isopentyldiol | 7,000 % |
| Isosorbide dimétyléther | 6,000 % |
| Eau distillée | 72,000 % |

Le mélange de tensioactif A est préparé en mélangeant 40-60 % en poids de trideceth-9, 20-40 % en poids de PEG 40 huile de ricin hydrogénée et jusqu'à 5 % en poids de Polysorbate-20.

### Exemple comparatif 2 : Composition parfumée comparative B

Les composants de la composition parfumée comparative B, décrits dans le tableau 17, sont mélangés afin d'obtenir la composition parfumée comparative B.

**Tableau 17**

| Désignation du produit | % En poids Quantité |
|---|---|
| Parfum 1 | 20,000 % |
| Hexanediol | 20,000 % |
| Eau distillée | 59,250 % |
| Phénoxyéthanol, Chlorphénésine, Caprylyl Glycol | 0,750 % |
| Siméthicone | 1×10⁻³ % |

### Exemple comparatif 3 : Composition parfumée comparative C

Les composants de la composition parfumée comparative C, décrits dans le tableau 18, sont mélangés afin d'obtenir la composition parfumée comparative C.

**Tableau 18**

| Désignation du produit | % En poids Quantité |
|---|---|
| Isopentyl acétate | 0,002 % |
| Méthyl Phenylacétate | 0,060 % |
| (Z)-Hex-3-enyl acétate | 0,015 % |
| 3,7-Diméthyl octa-1,6-diene-3-yl acétate | 0,300 % |
| 3-Méthyl-2-butenyl acétate | 0,002 % |
| Benzènemethanol, a-Methyl-, Acétate | 0,015 % |
| Undecanal | 0,001 % |
| Undecan-4-olide | 0,002 % |
| Benzènepropanal, .alpha.-méthyl-4-(1-méthylethyl)- | 0,001 % |
| (3aR-(3aalpha,5abeta,9aalpha,9bbeta))-Dodecahydro-3a,6,6,9a-tetraméthylnaphtho(2,1-b)furane | 0,005 % |
| Méthyl 2-aminobenzoate | 0,005 % |
| 2-Phényléthanol | 0,100 % |
| cis-Hex-3-en-1-ol | 0,015 % |
| 4-Méthyl-3-decen-5-ol | 0,010 % |
| 7-Méthyl-2H-benzo-1,5-dioxepin-3(4H)-one | 0,005 % |
| 2-Propényl (cyclohexyloxy)acétate | 0,005 % |
| Decan-5-olide | 0,001 % |
| 1-(2,6,6-Trimethyl-1,3-cyclohexadienyl)-2-buten-1-one | 0,003 % |
| 2,6-diméthyloct-7-en-2-ol | 0,002 % |
| 1,1'-Oxydipropan-2-ol | 6,327 % |
| 3,7-Diméthyl-1,6-nonadien-3-ol | 0,200 % |
| Huile de citron (Citrus Limon Fruit Oil) | 0,100 % |
| Méthyl hydroxypyrone | 0,001 % |
| 2,2,5-Triméthyl-5-pentylcyclopentan-1-one | 0,003 % |
| 3,4,4a,5,8,8a-Hexahydro-3',7'-dimethylspiro(1,4-méthanonaphthalène-2(1H),2'-oxirane) | 0,001 % |
| cis-2-Méthyl-4-propyl-1,3-oxathiane | 0,001 % |
| Méthyl 2,4-dihydroxy-3,6-diméthylbenzoate | 0,004 % |
| 2,4-diméthylcyclohex-3-ene-1-carbaldehyde | 0,006 % |
| méthyl 3-oxo-2-pentylcyclopentaneacetate | 2,402 % |
| Triéthyl citrate | 0,010 % |
| 1-(5,5-Diméthyl-1-cyclohexenyl)pent-4-en-1-one | 0,001 % |
| 3,7-Diméthyl-6-octen-1-ol | 0,001 % |
| 6,6-Diméthyl-2-methylènebicyclo[3.1.1]heptane | 0,002 % |
| 2,6,6-Triméthylbicyclo[3.1.1]hept-2-ene | 0,001 % |
| Huile de feuille de Pogostemon Cablin (Pogostemon Cablin Leaf Oil) | 0,005 % |
| Tétrahydro-4-méthyl-2-(2-méthylpropyl)-2H-pyran-4-ol | 0,650 % |
| 1,4-Dioxacycloheptadecane-5,17-dione | 0,750 % |
| Décan-4-olide | 0,005 % |
| alpha-Méthyl-1,3-benzodioxole-5-propionaldéhyde | 0,150 % |
| (E)-4-(2,6,6-triméthyl-1-cyclohexen-1-yl)-3-butèn-2-one | 0,200 % |
| 2-éthoxy-4-(méthoxyméthyl)-Phénol | 0,002 % |
| 3,7-Diméthyl octa-1,6-diène-3-ol | 0,250 % |
| 1,4-Dioxacyclohexadécane-5,16-Dione | 0,002 % |
| 3-Méthyl-5-cyclopentadecen-1-one | 0,010 % |
| Benzyle salicylate | 0,070 % |
| (Z)-3-Héxényl 2-hydroxybenzoate | 0,070 % |
| p-menth-1-en-8-ol | 0,001 % |
| 2-(1,1-Diméthyléthyl)Cyclohexyl Acétate | 0,003 % |
| Huile d'écorce de Citrus nobilis (Citrus nobilis peel oil) | 0,100 % |
| Huile d'écorce de Citrus Aurantium Dulcis (Citrus Aurantium Dulcis (Orange) Peel Oil) | 0,100 % |
| 2,4,6-Triméthyl-4-phényl-1,3-dioxane | 0,003 % |
| 4-(2,6,6-Triméthylcyclohex-2-eneyl)-but-3-ene-2-one | 0,020 % |
| C12-13 Pareth-9 | 4,000 % |
| Isopentyldiol | 15,000 % |
| Water | 68,250 % |
| 2-phénoxyethanol (et) Octane-1,2-diol (et) 3-(4-Chlorophénoxy)-1,2-propanediol | 0,750 % |

### Exemple comparatif 4 : Composition parfumée comparative D

Les composants de la composition parfumée comparative D, décrits dans le tableau 19, sont mélangés afin d'obtenir la composition parfumée comparative D.

**Tableau 19**

| Désignation du produit | % En poids Quantité |
|---|---|
| Méthyl Phenylacétate | 0,053 % |
| 3,7-Diméthyl octa-1 ,6-diene-3-yl acétate | 0,255 % |
| phénylméthanol | 0,135 % |
| Undecan-4-olide | 0,023 % |
| Huile d'herbe artemisia herba-alba (artemisia herba-alba herb oil) | 0,004 % |
| cis-Hex-3-en-1-ol | 0,002 % |
| Huile d'écorce de citron (Citrus limon (lemon) peel oil) | 0,975 % |
| Extrait de l'écorce de citronnier Citrus limon peel extract | 0,492 % |
| 6-Octenal, 3,7-diméthyl- | 0,002 % |
| 3,7-Diméthyl-6-octen-1-ol | 0,292 % |
| 1,1'-Oxydipropan-2-ol | 0,915 % |
| Géranyl acétate | 0,006 % |
| cis-3,7-Diméthyl-2,6-octadienyl éthanoate | 0,008 % |
| 2,6,6-Triméthylbicyclo[3.1.1]hept-2-ene | 0,011 % |
| 6,6-Dimethyl-2-methylenebicyclo[3.1.1]heptane | 0,407 % |
| 2,6-Octadien-1-ol, 3,7-diméthyl-, (2E)- | 0,000 % |
| cis-3,7-Diméthyl-2,6-octadien-1-ol | 0,026 % |
| 3,7-Diméthyl-1-octanol; | 0,001 % |
| 1-Méthyl-4-isopropenyl-1-cyclohexène | 0,009 % |
| 2,6-Octadienal, 3,7-diméthyl- | 1,053 % |
| 3,7-Diméthyl octa-1,6-diène-3-ol | 0,151 % |
| p-menth-1-en-8-ol | 0,009 % |
| 4-Méthyl-1-(1-méthyléthyl)-3-cyclohexen-1-ol | 0,002 % |
| 1-Méthyl-4-isopropyl-1,3-cyclohexadiène; | 0,072 % |
| Extrait d'écorce de Citrus aurantium dulcis (Citrus aurantium dulcis peel extract) | 3,916 % |
| Extrait de feuille d' Eucalyptus globulus (Eucalyptus globulus Leaf Extract) | 0,001 % |
| p-Menth-1-en-8-ol | 0,004 % |
| 2,6-Octadien-1-ol, 3,7-diméthyl-, (2E)- | 1,351 % |
| 2-(Tétrahydro-5-methyl-5-vinyl-2-furyl) propan-2-ol | 0,002 % |
| Huile de fruit Litsea cubeba (Litsea cubeba fruit oil) | 1,688 % |
| Huile de Myrocarpus Fastigiatus (Myrocarpus Fastigiatus Oil) | 0,001 % |
| Huile de fruit de Coriandrum sativum (Coriandrum sativum fruit oil) | 0,001 % |
| Huile de fleur de Pelargonium graveolens (Pelargonium graveolens flower oil) | 0,375 % |
| méthyl 3-oxo-2-pentylcyclopentaneacétate | 1,875 % |
| Indole | 0,012 % |
| 2-Phénylméthyleneoctanal | 0,690 % |
| cis-Hex-3-enyl méthyl carbonate | 0,006 % |
| Huile d'écorce de Citrus nobilis (Citrus nobilis peel oil) | 0,018 % |
| 6-Méthylhept-5-en-2-one | 0,001 % |
| 1-Méthyl-4-(1-methylethyl)benzène | 0,120 % |
| Huile de feuille de Citrus aurantium amara (Citrus aurantium amara leaf oil) | 0,026 % |
| Huile de feuille de Citrus limonum (Citrus limonum leaf oil) | 0,012 % |
| C12-13 Pareth-9 | 15,000 % |
| Isopentyldiol | 4,000 % |
| Water | 65,250 % |
| 2-phénoxyethanol (et) Octane-1,2-diol (et) 3-(4-Chlorophénoxy)-1,2-propanediol | 0,750 % |

### Epreuve de profil

### Préparation du test

L'objectif du test est de mettre en évidence les ressemblances et les différences entre produits sous forme, notamment de « cartes sensorielles » avec 32 panélistes.

Au cours de ce test, 3 descripteurs du parfum ont été évalués : « glissant », « absorption » et « intensité parfum ».

Pour chaque descripteur, un protocole précis a été donné aux panélistes afin que tous les panélistes évaluent de manière identique ces derniers.

Il a également été demandé aux panélistes de se laver les mains entre chaque référence testée avec du gel hydro alcoolique fourni afin d'éviter toute contamination croisée.

Les références évaluées ont été codées afin d'évaluer les produits en aveugle.

Par « glissant » : on entend l'évaluation de la propriété physique de résistance aux frottements du produit.

Pour ce test, une goutte de la composition à étudier est placée sur l'index du panéliste et un mouvement de frottement entre l'index et le pouce du panéliste est effectué. On évalue ensuite si le frottement s'effectue plus ou moins bien. Le résultat est reporté sur une échelle allant de 3 (très bien), i.e. le mouvement est facile, il n'y a pas de résistance à 0 (non-acceptable), i.e. le mouvement est difficile, il y a une forte résistance.

Par « absorption » : on entend l'évaluation de la propriété physique de pénétration du produit.

Pour ce test, une goutte de la composition à étudier est placée sur l'intérieur de l'avant-bras du panéliste, et la goutte est étalée en effectuant 15 tours de main (15 glissements). On évalue ensuite si la pénétration s'effectue plus ou moins bien. Le résultat est reporté sur une échelle allant de 3 (très bien), i.e. la pénétration est bonne et il n'y a aucun résidu de produit, à 0 (non-acceptable), i.e. la pénétration est mauvaise et il y a un résidu de produit.

Par « intensité parfum » : on entend l'évaluation de la propriété olfactive du produit après l'application.

Suite à l'étalement du produit effectué précédemment, les panélistes ont placé leurs nez à quelques centimètres de leurs peaux, et le niveau d'appréciation de l'intensité olfactive du parfum est évalué. Le résultat est reporté sur une échelle allant de 3 (très bien), i.e. bonne perception et parfum Intense, à 0 (non-acceptable), i.e. mauvaise perception et parfum trop faible.

Seulement 2 références sont évaluées par test, l'une sur le bras gauche avec application du produit avec la main droite et le second produit sur le bras droit avec application du produit avec la main gauche.

### Résultats

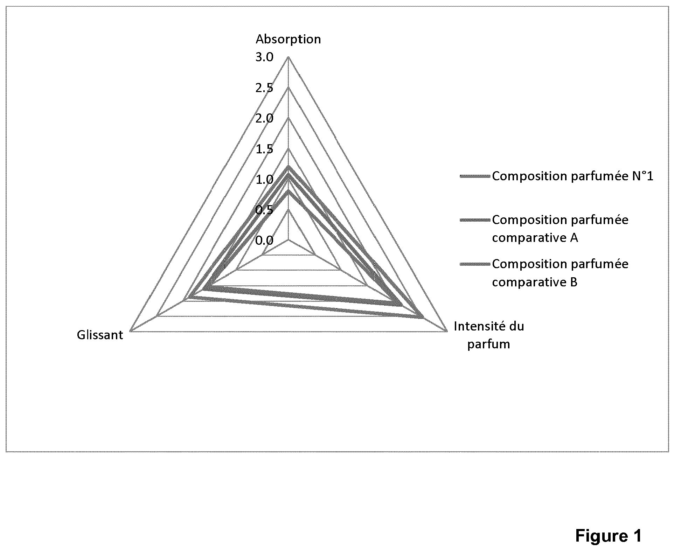

Les résultats, allant de 0 à 3 par pas de 0,5, sont présentés sur la figure 1.

La composition selon la présente invention (composition parfumée No. 1) présente une pénétration, intensité du parfum et résistance aux frottements supérieures aux compositions comparatives A et B étudiées.

Ainsi, la composition selon la présente invention est significativement meilleure que les compositions parfumées comparatives pour les descripteurs absorption, glissement et intensité du parfum.

### Tests additionnels

Après avoir comparé la transparence de la composition parfumée No. 1 avec celle de la composition comparative B, les présents inventeurs ont démontré que l'utilisation de diol vicinal (voir composition comparative B) rend la solution opaque. Au contraire, la composition parfumée No. 1 selon l'invention, dépourvue de diol vicinal, est transparente.

Après avoir comparé la transparence des compositions parfumées No. 1 à 6 avec celle de la composition comparative C, les présents inventeurs ont démontré que l'utilisation de C12-13 pareth-9 en une quantité inférieure à 5 % en poids par rapport au poids total de la composition parfumée rend la composition parfumée opaque.

Après avoir comparé la transparence des compositions parfumées No. 1 à 6 avec celle de la composition comparative D, les présents inventeurs ont démontré que l'utilisation de l'isopentyldiol en une quantité inférieure à 5 % en poids par rapport au poids total de la composition parfumée rend la composition opaque. En particulier, en utilisant de l'isopentyldiol en une quantité inférieure à 5 % en poids par rapport au poids total de la composition parfumée, la composition parfumée se trouble et devient opaque.

Il a également été démontré que les compositions parfumées selon la présente invention (voir compositions parfumées No. 1 à 6) sont transparentes dans des conditions de température allant de 4 à 50°C, quel que soit le parfum utilisé (y compris un parfum contenant moins de 18 % en poids d'alcool ramifié avec au minimum 8 atomes de carbone), à des dosages allant de 5 à 40 %.

Après avoir étudié l'intensité de parfum des compositions parfumées No. 1 à 6, il a été démontré qu'une composition selon l'invention présentait un degré olfactif supérieur à celui des compositions comparatives A, B, C et D.

Les compositions parfumées No. 1 à 6 sont peu collantes et peu moussantes, ne s'évaporent pas trop rapidement et présentent une solubilité et une performance olfactive améliorées en comparaison avec les compositions comparatives A, B, C et D.

### Exemple comparatif 5 : Comparaison C12-13 Pareth-9 contre Barsolve Plus

L'efficacité du C12-13 Pareth-9 a été testée contre celle du Barsolve Plus, mélange de 40-60% Trideceth-9, 20-40% PEG-40 Hydrogenated Castor oil, 5% Polysorbate-20, commercialisé par Barnet (Tableau 20).

Les ingrédients de la phase (A) sont tous ajoutés à température ambiante dans un bécher sous agitation. Après homogénéisation, le parfum (B) est ajouté sous faible agitation constante. Le parfum ici testé, en phase (B), est composé de 32% en poids d'alcools ramifiés avec au minimum 8 atomes de carbone (mélange de citronellol, dihydromyrcenol, ethyl linalool et linalool).

**Tableau 20 Tests comparatifs de l'efficacité du C12-13 Pareth-9 contre le Barsolve Plus (Cité dans WO2014/187950)**

| **Matières premières** | Essai 1 | Essai 2 |
|---|---|---|
| Parfum (B) | 7.00 | 7.00 |
| Isopentyldiol (A) | 7.00 | 7.00 |
| C12-13 Pareth-9 (A) | 8.00 | - |
| Barsolve Plus (A) | - | 8.00 |
| Eau déminéralisée (A) | 78.00 | 78.00 |
| **Résultat à température ambiante** | C | D |

| | | |
|---|---|---|
| Légende : C = clair, transparent V = voilé, bleu T = turbide, trouble D = déphasé | | |

Au regard des résultats ci-dessus, après comparaison de l'essai 1 avec l'essai 2, à même dosage de parfum, d'isopentyldiol et de solubilisant, nous observons que le C12-13 Pareth-9 permet d'obtenir une solution claire à température ambiante contrairement au Barsolve Plus lorsqu'il est utilisé sans co-solvant. Le solubilisant C12-13 Pareth-9 a donc un meilleur pouvoir de solubilisation que le Barsolve Plus. On en conclut donc que, contrairement au C12-13 Pareth-9, le Barsolve Plus n'est pas efficace sans co-solvant. Le C12-13 Pareth-9 est efficace sans co-solvant supplémentaire.

Des essais ont ensuite été réalisés à plus hauts dosages de parfum, d'isopentydiol et de solubilisant (Tableau 21). Il a également été testé l'ajout d'un co-solvant, Isosorbide dimethylether, au Barsolve Plus.

Comme les solutions obtenues sont toutes claires, des tests de stabilité sont effectués dans les conditions suivantes : 3 jours au réfrigérateur à 5°C, 3 jours en armoire thermostatée (respectivement à 40°C, 45°C et 50°C) et 24h sous UV (test réalisé avec un appareil Q-SUN Xe-1-B de Labomat, lampe Xénon, sous une longueur d'onde de 420nm, avec une irradiance de 0.62 W/m²).

**Tableau 21 Essais comparatifs C12-13 Pareth-9 contre Barsolve Plus à hauts dosages**

| **Matières premières** | Essai 3 | Essai 4 | Essai 5 |
|---|---|---|---|
| Parfum (B) | 10.00 | 10.00 | 10.00 |
| Isopentyldiol (A) | 16.00 | 16.00 | 16.00 |
| C12-13 Pareth-9 (A) | 9.00 | - | - |
| Barsolve Plus (A) | - | 9.00 | 9.00 |
| Isosorbide dimethylether (A) | - | - | 6.00 |
| Eau déminéralisée (A) | 65.00 | 65.00 | 59.00 |

| **Tests de stabilité** | Essai 3 | Essai 4 | Essai 5 |
|---|---|---|---|
| 5°C | C | D | D |
| TA | C | C | C |
| 40°C | C | C | C |
| 45°C | C | C | C |
| 50°C | C | C | C |
| UV | C | V | C |

Au regard des résultats ci-dessus, après comparaison de l'essai 3 avec l'essai 4, nous observons que le C12-13 Pareth-9 permet d'obtenir une meilleure stabilité à 5°C et aux UV. On en conclut donc que, contrairement au C12-13 Pareth-9, le Barsolve Plus ne permet pas d'obtenir une solution stable à 5°C, ni aux UVs lorsqu'il est utilisé sans co-solvant, lorsque le dosage de parfum est de 10%. Le C12-13 Pareth-9 permet d'obtenir une solution stable à 10% de parfum, dans toutes les conditions de tests, sans co-solvant supplémentaire.

Au regard des résultats ci-dessus, après comparaison de l'essai 3 avec l'essai 5, nous observons que le C12-13 Pareth-9 permet d'obtenir une meilleure stabilité à 5°C. On en conclut donc que contrairement au C12-13 Pareth-9, le Barsolve Plus ne permet pas d'obtenir une solution stable à 5°C même lorsqu'il est utilisé avec un co-solvant, lorsque le dosage de parfum est de 10%. Le C12-13 Pareth-9 permet d'obtenir une solution stable à 10% de parfum dans toutes les conditions de tests, sans co-solvant supplémentaire.

### Exemple comparatif 6 : Comparaison des quantités d'isopentydiol nécessaires pour obtenir des solutions claires à température ambiante

Des essais ont été réalisés au même dosage de parfum et de solubilisant, afin de déterminer la quantité d'isopentyldiol nécessaire pour obtenir des solutions claires à température ambiante (Tableau 22).

**Tableau 22 Essais comparatifs des quantités d'isopentydiol nécessaires**

| **Matières premières** | Essai 3 | Essai 6 | Essai 7 | Essai 4 | Essai 8 | Essai 9 | Essai 5 | Essai 10 | Essai 11 |
|---|---|---|---|---|---|---|---|---|---|
| Parfum (B) | 10.00 | 10.00 | 10.00 | 10.00 | 10.00 | 10.00 | 10.00 | 10.00 | 10.00 |
| Isopentyldiol (A) | 16.00 | 12.00 | 10.00 | 16.00 | 12.00 | 10.00 | 16.00 | 12.00 | 10.00 |
| C12-13 Pareth-9 (A) | 9.00 | 9.00 | 9.00 | - | - | - | - | - | - |
| Barsolve Plus | - | - | - | 9.00 | 9.00 | 9.00 | 9.00 | 9.00 | 9.00 |
| Isosorbide dimethylether (A) | - | - | - | - | - | - | 6.00 | 6.00 | 6.00 |
| Eau déminéralisée (A) | 65.00 | 69.00 | 71.00 | 65.00 | 69.00 | 71.00 | 59.00 | 63.00 | 65.00 |
| **Résultat à température ambiante** | C | C | C | C | D | T | C | C | T |

Au regard des résultats ci-dessus, après comparaison des essais 3, 6 et 7, le C12-13 Pareth-9 permet d'obtenir des solutions claires à température ambiante avec des quantités d'isopentydiol de 16%, 12% et même 10%.

Au regard des résultats ci-dessus, après comparaison des essais 4, 8 et 9, le Barsolve Plus, utilisé sans co-solvant, permet d'obtenir des solutions claires à température ambiante uniquement avec une quantité d'isopentyldiol de 16%. On en conclut donc que, le Barsolve Plus utilisé sans co-solvant, nécessite une plus grande quantité d'isopentyldiol que le C12-13 Pareth-9 pour obtenir des solutions claires à température ambiante.

Au regard des résultats ci-dessus, après comparaison des essais 5, 10 et 11, le Barsolve Plus, utilisé avec co-solvant, permet d'obtenir des solutions claires à température ambiante avec des quantités d'isopentyldiol de 16% et 12%. On en conclut donc que, le Barsolve Plus utilisé avec co-solvant, nécessite une plus grande quantité d'isopentyldiol que le C12-13 Pareth-9 pour obtenir des solutions claires à température ambiante.

Le C12-13 Pareth-9 nécessite donc moins d'isopentyldiol et ne nécessite pas d'autre co-solvant pour obtenir des solutions claires et stables.

## Revendications

1. Complexe parfumant ne comprenant pas d'eau et comprenant trois composants de base suivants : 15 à 45 % en poids de parfum, 15 à 50 % en poids d'isopentyldiol et 15 à 30 % en poids de C12-13 pareth-9, les pourcentages étant exprimés par rapport au poids total des trois composants de base.

2. Complexe parfumant selon la revendication précédente étant exempt d'éthanol.

3. Complexe parfumant selon l'une quelconque des revendications précédentes constitué des trois composants de bases.

4. Complexe parfumant selon l'une quelconque des revendications précédentes comprenant 20 à 40 % en poids de parfum.

5. Complexe parfumant selon l'une quelconque des revendications précédentes comprenant de 27 % à 31 % en poids de parfum.

6. Complexe parfumant selon l'une quelconque des revendications précédentes comprenant 20 à 30 % en poids de C12-13 pareth-9.

7. Complexe parfumant selon l'une quelconque des revendications précédentes comprenant 24 à 29 % en poids de C12-13 pareth-9.

8. Complexe parfumant selon l'une quelconque des revendications précédentes comprenant 35 % à 50 % en poids d'isopentyldiol.

9. Complexe parfumant selon l'une quelconque des revendications précédentes comprenant 42 % à 47 % en poids d'isopentyldiol.

10. Utilisation d'un complexe parfumant selon l'une quelconque des revendications 1 à 9 dans un milieu aqueux.

11. Composition comprenant le complexe parfumant selon l'une quelconque des revendications précédentes et de l'eau et ne contenant aucun co-solvant du groupe comprenant l'isosorbide, le solketal et des éthers de ceux-ci.

12. Composition selon la revendication précédente étant une composition parfumée sous forme de microémulsion.

13. Composition selon l'une quelconque des deux revendications précédentes, étant exempte d'éthanol.

14. Composition selon l'une quelconque des trois revendications précédentes comprenant de 30 à 40 % en poids de complexe parfum par rapport au poids total de la composition.

15. Composition selon l'une quelconque des revendications 11 à 14 comprenant 5 à 40 % en poids de parfum, 5 à 30 % en poids C12-13 pareth-9, et 5 à 30 % en poids d'isopentyldiol, les pourcentages étant exprimés par rapport au poids total de ladite composition.

16. Composition selon l'une quelconque des revendications 11 à 15, **caractérisée en ce qu'**elle comprend en outre au moins un additif choisi parmi les agents anti-mousses, agents antioxydants, agents chélatants, filtres UV, conservateurs, agents épaississants, ingrédients actifs cosmétiques, agents hydratants, humectants, adoucissants, pigments, colorants, agent réfrigérant, agents ajusteurs de pH, agents bactéricides, agents bactériostatiques, insecticides, agents répulsifs et leurs mélanges.

17. Composition selon la revendication précédente, **caractérisée en ce que** les additifs sont présents en une quantité allant de 0 à 5 %, de préférence de 0,001 à 2 %, plus préférentiellement de 0,05 à 1 %, et encore plus préférentiellement de 0,001 à 0,1 % en poids par rapport au poids total de ladite composition.

18. Base cosmétique, base détergente ou parfum d'ambiance comprenant la composition telle que définie selon l'une quelconque des revendications 11 à 17.

19. Utilisation de la composition telle que définie selon l'une quelconque des revendications 11 à 17, de la base cosmétique, de la base détergente ou du parfum d'ambiance tels que définis à la revendication 18 pour masquer les mauvaises odeurs.

## Patentansprüche

1. Parfümkomplex, der kein Wasser umfasst und die folgenden drei Basiskomponenten umfasst: 15 bis 45 Gew.-% Parfüm, 15 bis 50 Gew.-% Isopentyldiol und 15 bis 30 Gew.-% C12-13 Pareth-9, wobei sich die Prozentanteile auf das Gesamtgewicht der drei Basiskomponenten beziehen.

2. Parfümkomplex nach dem vorhergehenden Anspruch, der frei von Ethanol ist.

3. Parfümkomplex nach einem der vorhergehenden Ansprüche, der aus den drei Basiskomponenten besteht.

4. Parfümkomplex nach einem der vorhergehenden Ansprüche, der 20 bis 40 Gew.-% Parfüm umfasst.

5. Parfümkomplex nach einem der vorhergehenden Ansprüche, der 27 bis 31 Gew.-% Parfüm umfasst.

6. Parfümkomplex nach einem der vorhergehenden Ansprüche, der 20 bis 30 Gew.-% von C12-13 Pareth-9 umfasst.

7. Parfümkomplex nach einem der vorhergehenden Ansprüche, der 24 bis 29 Gew.-% von C12-13 Pareth-9 umfasst.

8. Parfümkomplex nach einem der vorhergehenden Ansprüche, der 35 bis 50 Gew.-% Isopentyldiol umfasst.

9. Parfümkomplex nach einem der vorhergehenden Ansprüche, der 42 bis 47 Gew.-% Isopentyldiol umfasst.

10. Verwendung eines Parfümkomplexes nach einem der Ansprüche 1 bis 9 in einem wässrigen Medium.

11. Zusammensetzung, die den Parfümkomplex nach einem der vorhergehenden Ansprüche und Wasser umfasst und kein Co-Lösungsmittel aus der Gruppe enthält, die Isosorbid, Solketal und Ether davon umfasst.

12. Zusammensetzung nach dem vorhergehenden Anspruch, die eine parfümierte Zusammensetzung in Mikroemulsionsform umfasst.

13. Zusammensetzung nach einem der beiden vorhergehenden Ansprüche, die frei von Ethanol ist.

14. Zusammensetzung nach einem der drei vorhergehenden Ansprüche, die 30 bis 40 Gew.-% des Parfümkomplexes bezogen auf das Gesamtgewicht der Zusammensetzung umfasst.

15. Zusammensetzung nach einem der Ansprüche 11 bis 14, die 5 bis 40 Gew.-% Parfüm, 5 bis 30 Gew.-% C12-13 Pareth-9 und 5 bis 30 Gew.-% Isopentyldiol umfasst, wobei sich die Prozentanteile auf das Gesamtgewicht der drei Basiskomponenten beziehen.

16. Zusammensetzung nach einem der Ansprüche 11 bis 15, **dadurch gekennzeichnet, dass** sie ferner mindestens einen Zusatzstoff umfasst, der unter Antischaummitteln, Antioxidantien, Chelatbildnern, UV-Filtern, Konservierungsmitteln, Verdickungsmitteln, kosmetischen Wirkstoffen, Feuchtigkeitsmitteln, Feuchthaltemitteln, Weichmachern, Pigmenten, Farbstoffen, Kühlmitteln, pH-Einstellungsmitteln, bakteriziden Mitteln, bakteriostatischen Mitteln, Insektiziden, Repellentien und Mischungen davon ausgewählt ist.

17. Zusammensetzung nach dem vorhergehenden Anspruch, **dadurch gekennzeichnet, dass** die Zusatzstoffe in einer Menge von 0 bis 5 Gew.-%, vorzugsweise von 0,001 bis 2 Gew.-%, noch bevorzugter 0,05 bis 1 Gew.-% und besonders bevorzugt 0,001 bis 0,1 Gew.-%, bezogen auf das Gesamtgewicht der Zusammensetzung, vorhanden sind.

18. Kosmetische Basis, Waschmittelbasis oder Raumparfüm, die bzw. das die Zusammensetzung umfasst, wie sie in einem der Ansprüche 11 bis 17 definiert ist.

19. Verwendung der Zusammensetzung, wie sie in einem der Ansprüche 11 bis 17 definiert ist, der Kosmetikbasis, der Waschmittelbasis oder des Raumparfums, wie sie in Anspruch 18 definiert sind, zum Überdecken von unangenehmen Gerüchen.

## Claims

1. Perfume complex not comprising water and comprising three basic ingredients as follows: 15 to 45 wt.% perfume, 15 to 50 wt.% isopentyldiol and 15 to 30 wt.% C12-13 pareth-9, the percentages being expressed with respect to the total weight of the three basic ingredients.

2. Perfume complex according to the preceding claim being free from ethanol.

3. Perfume complex according to any one of the preceding claims consisting of the three basic ingredients.

4. Perfume complex according to any one of the preceding claims comprising 20 to 40 wt.% perfume.

5. Perfume complex according to any one of the preceding claims comprising 27 to 31 wt.% perfume.

6. Perfume complex according to any one of the preceding claims comprising 20 to 30 wt.% C12-13 pareth-9.

7. Perfume complex according to any one of the preceding claims comprising 24 to 29 wt.% C12-13 pareth-9.

8. Perfume complex according to any one of the preceding claims comprising 35 to 50 wt.% isopentyldiol.

9. Perfume complex according to any one of the preceding claims comprising 42 to 47 wt.% isopentyldiol.

10. Use of a perfume complex according to any one of claims 1 to 9 in an aqueous medium.

11. Composition comprising the perfume complex according to any one of the preceding claims and water and not containing any co-solvent of the group comprising isosorbide, solketal and ethers thereof.

12. Composition according to the preceding claim being a perfumed composition in microemulsion form.

13. Composition according to any one of the two preceding claims, being free from ethanol.

14. Composition according to any one of the three preceding claims comprising 30 to 40 wt.% perfume complex with respect to the total weight of the composition.

15. Composition according to any one of claims 11 to 14 comprising 5 to 40 wt.% perfume, 5 to 30 wt.% C12-13 pareth-9, and 5 to 30 wt.% isopentyldiol, the percentages being expressed with respect to the total weight of said composition.

16. Composition according to any one of claims 11 to 15, **characterised in that** it further comprises at least one additive selected from anti-foaming agents, antioxidant agents, chelating agents, UV filters, preservatives, thickening agents, cosmetic active ingredients, hydrating agents, humectants, demulcents, pigments, colorants, cooling agent, pH-adjusting agents, bactericidal agents, bacteriostatic agents, insecticides, repellents and mixtures thereof.

17. Composition according to the preceding claim, **characterised in that** the additives are present in an amount ranging from 0 to 5 wt.%, preferably from 0.001 to 2%, more preferably from 0.05 to 1%, and even more preferably from 0.001 to 0.1% by weight with respect to the total weight of said composition

18. Cosmetic base, detergent base or ambient perfume comprising the composition as defined according to any one of claims 11 to 17.

19. Use of the composition as defined according to any one of claims 11 to 17, of the cosmetic base, of the detergent base or of the ambient perfume as defined in claim 18 to mask unpleasant odours.
